# EUROPÄISCHE PATENTSCHRIFT

(11) **EP 0 610 697 B1**
(45) Veröffentlichungstag und Bekanntmachung des Hinweises auf die Patenterteilung: **12.03.1997**
(21) Anmeldenummer: 94100845.0
(22) Anmeldetag: 21.01.1994
(51) Int. Cl.: C07D 403/10, C07K 5/06, A61K 31/415

(54) **Imidazolyl-substituierte Phenylessigsäureprolinamide**
Imidazole substituted phenylaceticacidprolinzamide derivatives
Dérivés imidazolique de l'acide phénylacétique prolinamide

(30) Priorität: 03.02.1993 DE 4302957
(43) Veröffentlichungstag der Anmeldung: 17.08.1994
(73) Patentinhaber: BAYER AG, 51368 Leverkusen (DE)
(72) Erfinder: Müller-Gliemann, Matthias, Dr., D-42697 Solingen (DE); Dressel, Jürgen, Dr., D-42477 Radevormwald (DE); Fey, Peter, Dr., D-42111 Wuppertal (DE); Hanko, Rudolf H., Dr., D-40237 Düsseldorf (DE); Hübsch, Walter, Dr., D-42113 Wuppertal (DE); Krämer, Thomas, Dr., D-42111 Wuppertal (DE); Müller, Ulrich E., Dr., D-42111 Wuppertal (DE); Beuck, Martin, Dr., D-40699 Erkrath (DE); Kazda, Stanislav, Prof., Dr., D-42115 Wuppertal (DE); Wohlfeil, Stefan, Dr., D-40724 Hilden (DE); Knorr, Andreas, Dr., D-40699 Erkrath (DE); Stasch, Johannes-Peter, Dr., D-42651 Solingen (DE); Zaiss, Siegfried, Dr., D-42113 Wuppertal (DE)

(56) Entgegenhaltungen:
- EP-A- 0 010 347
- EP-A- 0 513 533
- EP-A- 0 560 163
- EP-A- 0 573 271
- WO-A-91/12002
- J. MED. CHEM. Bd. 33 , 1990 Seiten 1312 - 1329 J.V.DUNCIA ET AL 'The discovery of potent nonpeptide angiotensin II receptor antagonists: A new class of potent antihypertensives.'

## Beschreibung

Die Erfindung betrifft Imidazolyl-substituierte Phenylessigsäureprolinamide, Verfahren zu ihrer Herstellung sowie ihre Verwendung in Arzneimitteln, insbesondere als blutdrucksenkende und antiatherosklerotische Mittel.

Es ist bekannt, daß Renin, ein proteolytisches Enzym, in vivo vom Angiotensinogen das Dekapeptid Angiotensin I abspaltet, welches wiederum in der Lunge, den Nieren oder anderen Geweben zu dem blutdrucksteigerndes Oktapeptid Angiotensin II abgebaut wird. Die verschiedenen Effekte des Angiotensin II, wie beispielsweise Vasokonstriktion, Na⁺-Retention in der Niere, Aldosteronfreisetzung in der Nebenniere und Tonuserhöhung des sympathischen Nervensystems wirken synergistisch im Sinne einer Blutdruckerhöhung.

Darüber hinaus besitzt Angiotensin II die Eigenschaft, das Wachstum und die Vermehrung von Zellen wie beispielsweise von Herzmuskelzellen und glatten Muskelzellen zu fördern, wobei diese bei verschiedenen Krankheitszuständen (z.B. Hypertonie, Atherosklerose und Herzinsuffizienz) vermehrt wachsen und proliferieren.

Ein möglicher Ansatz zum Eingriff in das Renin-Angiotensin-System (RAS) ist neben der Inhibierung der Reninaktivität die Hemmung der Aktivität des Angiotensin-Konversionsenzyms (ACE) sowie die Blockade von Angiotensin II-Rezeptoren.

Die vorliegende Erfindung betrifft Imidazolyl-substituierte Phenylessigsäureprolinamide der allgemeinen Formel (I) 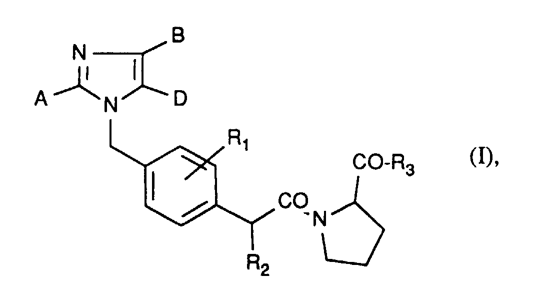 in welcher
- A: für geradkettiges oder verzweigtes Alkyl oder Alkenyl mit jeweils bis zu 8 Kohlenstoffatomen steht, oder
für Cycloalkyl mit 3 bis 8 Kohlenstoffatomen steht,
- B: für Wasserstoff, Halogen oder für Perfluoralkyl mit bis zu 5 Kohlenstoffatomen steht,
- D: für eine Gruppe der Formel-CH₂-OR⁴ oder -CO-R⁵ steht,
worin
R⁴ Wasserstoff oder geradkettiges oder verzweigtes Alkyl mit bis zu 8 Kohlenstoffatomen bedeutet,
R⁵ Wasserstoff, Hydroxy oder geradkettiges oder verzweigtes Alkoxy mit bis zu 8 Kohlenstoffatomen bedeutet,
- R¹: für Wasserstoff, Halogen, Nitro, Hydroxy, Trifluormethyl, Trifluormethoxy, geradkettiges oder verzweigtes Alkyl, Alkoxy oder Alkoxycarbonyl mit jeweils bis zu 6 Kohlenstoffatomen, Cyano oder Carboxy steht,
- R²: für Wasserstoff, geradkettiges oder verzweigtes Alkyl mit bis zu 8 Kohlenstoffatomen oder Cycloalkyl mit 3 bis 8 Kohlenstoffatomen steht,
- R³: für Hydroxy, Benzyloxy oder für geradkettiges oder verzweigtes Alkoxy mit bis zu 8 Kohlenstoffatomen steht, oder
für eine Gruppe der Formel
-NR⁶R⁷ oder 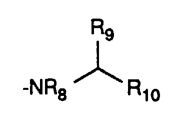 steht,
worin
R⁶, R⁷ und R⁸ gleich oder verschieden sind und Wasserstoff oder geradkettiges oder verzweigtes Alkyl mit bis zu 8 Kohlenstoffatomen bedeuten, oder
R⁶ die oben angegebene Bedeutung hat und
R⁷ einen Rest der Formel -SO₂R¹¹ bedeutet,
worin
R¹¹ geradkettiges oder verzweigtes Alkyl mit bis zu 4 Kohlenstoffatomen bedeutet, das gegebenenfalls durch Phenyl oder Cycloalkyl mit 3 bis 6 Kohlenstoffatomen substituiert ist, oder
Phenyl bedeutet, das gegebenenfalls durch geradkettiges oder verzweigtes Alkyl mit bis zu 4 Kohlenstoffatomen substituiert ist,
R⁹ Phenyl bedeutet, das gegebenenfalls bis zu 2-fach gleich oder verschieden durch Halogen, Nitro, Hydroxy, Trifluormethyl, Trifluormethoxy, geradkettiges oder verzweigtes Alkyl, Alkoxy oder Alkoxycarbonyl mit jeweils bis zu 6 Kohlenstoffatomen, Cyano, Carboxy, Cycloalkyl mit 3 bis 6 Kohlenstoffatomen oder Phenyl substituiert ist,
R¹⁰ Carboxy oder geradkettiges oder verzweigtes Alkoxycarbonyl mit bis zu 8 Kohlenstoffatomen bedeutet, oder
einen Rest der Formel -CH₂OR¹², -CO-NR¹³R¹⁴ oder -CH₂NR¹³R¹⁴ bedeutet,
worin
R¹² Wasserstoff, Benzyl oder geradkettiges oder verzweigtes Alkyl mit bis zu 8 Kohlenstoffatomen bedeutet,
R¹³ und R¹⁴ gleich oder verschieden sind und Wasserstoff, Phenyl oder geradkettiges oder verzweigtes Alkyl mit bis zu 6 Kohlenstoffatomen bedeuten,
gegebenenfalls in einer isomeren Form und deren Salze.

Die erfindungsgemäßen Verbindungen der allgemeinen Formel (I) können auch in Form ihrer Salze vorliegen. Im allgemeinen seien hier Salze mit organischen oder anorganischen Basen oder Säuren genannt.

Im Rahmen der vorliegenden Erfindung werden physiologisch unbedenkliche Salze bevorzugt. Physiologisch unbedenkliche Salze der Imidazolyl-substituierten Phenylessigsäureprolinamide können Salze der erfindungsgemäßen Stoffe mit Mineralsäuren, Carbonsäuren oder Sulfonsäuren sein. Besonders bevorzugt sind z.B. Salze mit Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Propionsäure, Milchsäure, Weinsäure, Zitronensäure, Fumarsäure, Maleinsäure oder Benzoesäure.

Physiologisch unbedenkliche Salze können ebenso Metall- oder Ammoniumsalze der erfindungsgemäßen Verbindungen sein, welche eine freie Carboxylgruppe besitzen. Besonders bevorzugt sind z.B. Natrium-, Kalium-, Magnesium- oder Calciumsalze, sowie Ammoniumsalze, die abgeleitet sind von Ammoniak, oder organischen Aminen wie beispielsweise Ethylamin, Di- bzw. Triethylamin, Di- bzw. Triethanolamin, Dicyclohexylamin, Dimethylaminoethanol, Arginin, Lysin oder Ethylendiamin.

Die erfindungsgemäßen Verbindungen können in stereoisomeren Formen, die sich entweder wie Bild und Spiegelbild (Enantiomere), oder die sich nicht wie Bild und Spiegelbild (Diastereomere) verhalten, existieren. Die Erfindung betrifft sowohl die Enantiomeren oder Diastereomeren oder deren jeweiligen Mischungen. Die Racemformen lassen sich ebenso wie die Diastereomeren in bekannter Weise in die stereoisomer einheitlichen Bestandteile trennen.

Bevorzugt sind Verbindungen der allgemeinen Formel (I),
in welcher
- A: für geradkettiges oder verzweigtes Alkyl oder Alkenyl mit jeweils bis zu 6 Kohlenstoffatomen steht, oder
für Cyclopropyl, Cyclobutyl, Cyclopentyl, Cyclohexyl oder Cycloheptyl steht,
- B: für Wasserstoff, Fluor, Chlor, Brom oder für Perfluoralkyl mit bis zu 4 Kohlenstoffatomen steht,
- D: für eine Gruppe der Formel -CH₂OR⁴ oder -CO-R⁵ steht,
worin
R⁴ Wasserstoff oder geradkettiges oder verzweigtes Alkyl mit bis zu 6 Kohlenstoffatomen bedeutet,
R⁵ Wasserstoff, Hydroxy oder geradkettiges oder verzweigtes Alkoxy mit bis zu 6 Kohlenstoffatomen bedeutet,
- R¹: für Wasserstoff, Fluor, Chlor, Brom, Trifluormethyl, Cyano oder geradkettiges oder verzweigtes Alkyl, Alkoxy oder Alkoxycarbonyl mit jeweils bis zu 5 Kohlenstoffatomen steht,
- R²: für Wasserstoff, geradkettiges oder verzweigtes Alkyl mit bis zu 6 Kohlenstoffatomen, Cyclopentyl, Cyclohexyl oder Cycloheptyl steht,
- R³: für Hydroxy, Benzyloxy oder für geradkettiges oder verzweigtes Alkoxy mit bis zu 6 Kohlenstoffatomen steht, oder
für eine Gruppe der Formel
-NR⁶R⁷ oder 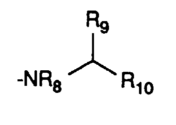 steht,
worin
R⁶, R⁷ und R⁸ gleich oder verschieden sind und Wasserstoff oder geradkettiges oder verzweigtes Alkyl mit bis zu 6 Kohlenstoffatomen bedeuten, oder
R⁶ die oben angegebene Bedeutung hat und
R⁷ einen Rest der Formel -SO₂R¹¹ bedeutet,
worin
R¹¹ geradkettiges oder verzweigtes Alkyl mit bis zu 3 Kohlenstoffatomen, Benzyl oder p-Tolyl bedeutet,
R⁹ Phenyl bedeutet, das gegebenenfalls durch Fluor, Chlor, Brom, Trifluormethyl, Carboxy oder geradkettiges oder verzweigtes Alkyl, Alkoxy oder Alkoxycarbonyl mit jeweils bis zu 4 Kohlenstoffatomen substituiert ist,
R¹⁰ Carboxy oder geradkettiges oder verzweigtes Alkoxycarbonyl mit bis zu 6 Kohlenstoffatomen bedeutet, oder
einen Rest der Formel -CH₂-OR¹², -CO-NR¹³R¹⁴ oder -CH₂NR¹³R¹⁴ bedeutet,
worin
R¹² Wasserstoff, Benzyl oder geradkettiges oder verzweigtes Alkyl mit bis zu 6 Kohlenstoffatomen bedeutet,
R¹³ und R¹⁴ gleich oder verschieden sind und Wasserstoff, Phenyl oder geradkettiges oder verzweigtes Alkyl mit bis zu 4 Kohlenstoffatomen bedeuten,
gegebenenfalls in einer isomeren Form und deren Salze.

Besonders bevorzugt sind Verbindungen der allgemeinen Formel (I),
in welcher
- A: für geradkettiges oder verzweigtes Alkyl oder Alkenyl mit jeweils bis zu 4 Kohlenstoffatomen steht, oder
für Cyclopropyl, Cyclopentyl oder Cyclohexyl steht,
- B: für Wasserstoff, Fluor, Chlor oder für Perfluoralkyl mit bis zu 2 Kohlenstoffatomen steht,
- D: für eine Gruppe der Formel -CH₂OR⁴ oder -CO-R⁵ steht,
worin
R⁴ Wasserstoff oder geradkettiges oder verzweigtes Alkyl mit bis zu 4 Kohlenstoffatomen bedeutet,
R⁵ Wasserstoff, Hydroxy oder geradkettiges oder verzweigtes Alkoxy mit bis zu 4 Kohlenstoffatomen bedeutet,
- R¹: für Wasserstoff, Fluor, Chlor, Brom, Trifluormethyl, Trifluormethoxy, Methyl oder Isobutyl steht,
- R²: für Wasserstoff, geradkettiges oder verzweigtes Alkyl mit bis zu 4 Kohlenstoffatomen, Cyclopentyl, Cyclohexyl oder Cycloheptyl steht,
- R³: für Hydroxy, Benzyloxy oder für geradkettiges oder verzweigtes Alkoxy mit bis zu 4 Kohlenstoffatomen steht, oder für eine Gruppe der Formel
-NR⁶R⁷ oder 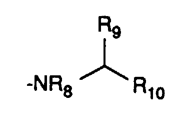 steht,
worin
R⁶, R⁷ und R⁸ gleich oder verschieden sind und Wasserstoff oder geradkettiges oder verzweigtes Alkyl mit bis zu 4 Kohlenstoffatomen bedeuten, oder
R⁶ die oben angegebene Bedeutung hat und
R⁷ einen Rest der Formel -SO₂R¹¹ bedeutet,
worin
R¹¹ Methyl, Benzyl oder p-Tolyl bedeutet,
R⁹ Phenyl bedeutet, das gegebenenfalls durch Fluor, Chlor, Brom, Trifluormethyl, Trifluormethoxy oder Methyl substituiert ist,
R¹⁰ Carboxy oder geradkettiges oder verzweigtes Alkoxycarbonyl mit bis zu 4 Kohlenstoffatomen bedeutet, oder einen Rest der Formel -CH₂-OR¹², -CO-NR¹³R¹⁴ oder -CH₂NR¹³R¹⁴ bedeutet,
worin
R¹² Wasserstoff, Benzyl oder geradkettiges oder verzweigtes Alkyl mit bis zu 4 Kohlenstoffatomen bedeutet,
R¹³ und R¹⁴ gleich oder verschieden sind und Wasserstoff oder geradkettiges oder verzweigtes Alkyl mit bis zu 3 Kohlenstoffatomen bedeuten,
gegebenenfalls in einer isomeren Form und deren Salze.

Ganz besonders bevorzugt sind Verbindungen der allgemeinen Formel (I),
in welcher
- R²: für Cyclopentyl oder Cycloheptyl steht.

Außerdem wurde ein Verfahren zur Herstellung der erfindungsgemäßen Verbindungen der allgemeinen Formel (I) gefunden, dadurch gekennzeichnet, daß man

Verbindungen der allgemeinen Formel (II) 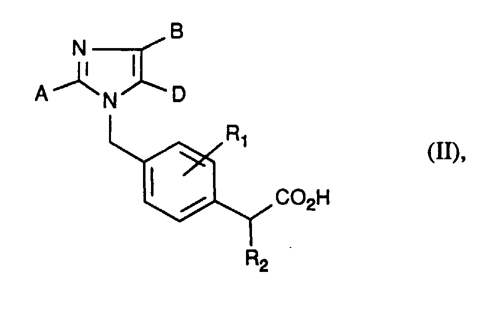 in welcher
A, B, D, R¹ und R² die oben angegebene Bedeutung haben,
mit Prolinderivaten der allgemeinen Formel (III) 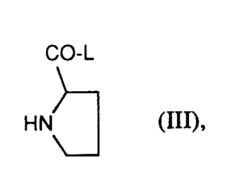 in welcher
- L: für geradkettiges oder verzweiges (C₁-C₄)-Alkoxy oder Benzyloxy steht,
in inerten Lösemitteln, in Anwesenheit einer Base und/oder eines Dehydratisierungsmittels umsetzt,
und im Fall der Prolinsäuren (R³ = OH) die Ester verseift,
und im Fall der Prolinamide (R⁶/R⁷ = H oder Alkyl) ausgehend von den Säuren, gegebenenfalls nach vorgeschalteter Aktivierung, wie oben beschrieben, mit Aminen der allgemeinen Formel (IV)

HNR⁶R⁷ (IV),

in welcher
R⁶ und R⁷ die oben angegebene Bedeutung haben,
umsetzt,
und im Fall der Prolin-Phenylglycinamide (-NR⁸-CHR⁹-R¹⁰) die Säuren, wie oben beschrieben, in inerten Lösemitteln in Anwesenheit einer Base und/oder eines Hilfsstoffes mit Verbindungen der allgemeinen Formel (V) 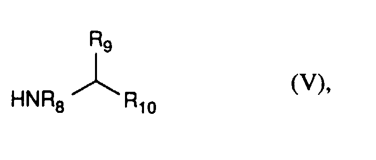 in welcher
R⁸, R⁹ und R¹⁰ die oben angegebene Bedeutung haben,
umsetzt,
und gegebenenfalls die Substituenten A, B, D und R¹ nach üblichen Methoden, beispielsweise durch Reduktion, Oxidation, Alkylierung oder Hydrolyse einführt oder in andere Gruppen überführt,
und gegebenenfalls die Isomeren trennt, und im Fall der Herstellung der Salze mit einer entsprechenden Base oder Säure umsetzt.

Das erfindungsgemäße Verfahren kann durch folgendes Formelschema beispielhaft erläutert werden:

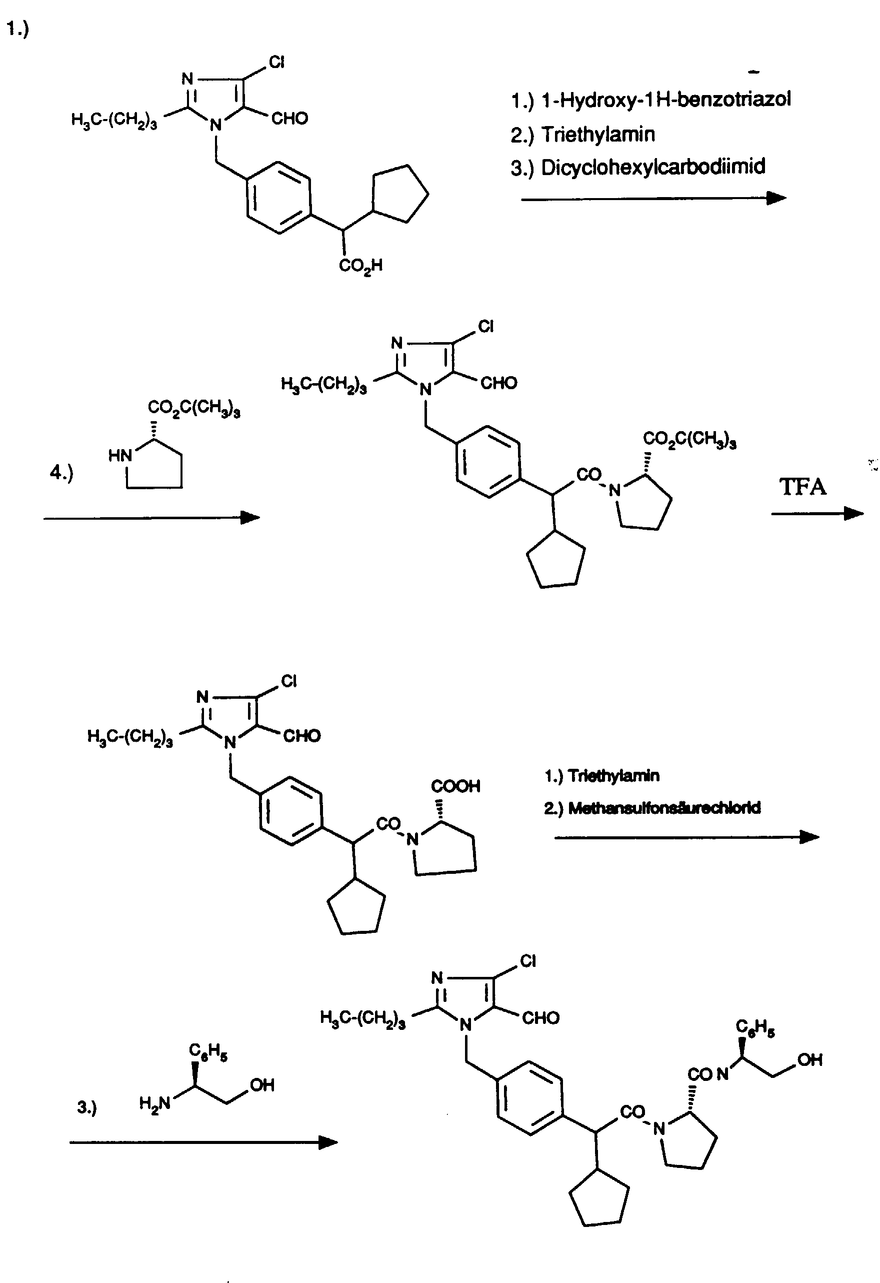

Als Lösemittel für das Verfahren eignen sich übliche organische Lösemittel, die sich unter den Reaktionsbedingungen nicht verändern. Hierzu gehören bevorzugt Ether wie Diethylether, Dioxan, Tetrahydrofuran, Glykoldimethylether, oder Kohlenwasserstoffe wie Benzol, Toluol, Xylol, Hexan, Cyclohexan oder Erdölfraktionen, oder Halogenkohlenwasserstoffe wie Dichlormethan, Trichlormethan, Tetrachlormethan, Dichlorethylen, Trichlorethylen oder Chlorbenzol, oder Essigester, Triethylamin, Pyridin, Dimethylsulfoxid, Dimethylformamid, Hexamethylphosphorsäuretriamid, Acetonitril, Aceton oder Nitromethan. Ebenso ist es möglich, Gemische der genannten Lösemittel zu verwenden. Bevorzugt sind Dimethylformamid und Tetrahydrofuran.

Als Basen für das erfindungsgemäße Verfahren können im allgemeinen anorganische oder organische Basen eingesetzt werden. Hierzu gehören vorzugsweise Alkalihydroxide lihydroxide wie zum Beispiel Natriumhydroxid oder Kaliumhydroxid, wie zum Beispiel Bariumhydroxid, Alkalicarbonate wie Natriumcarbonat, Kaliumcarbonat oder Cäsiumcarbonat, Erdalkalicarbonate wie Calciumcarbonat, oder Alkali- oder Erdalkalialkoholate wie Natrium- oder Kaliummethanolat, Natrium- oder Kaliumethanolat oder Kalium-tert.butylat, oder organische Amine (Trialkyl(C₁-C₆)amine) wie Triethylamin, oder Heterocyclen wie 1,4-Diazabicyclo[2.2.2]octan (DABCO), 1,8-Diazabicyclo[5.4.0]undec-7-en (DBU), Pyridin, Diaminopyridin, Methylpiperidin oder Morpholin. Es ist auch möglich, als Basen Alkalimetalle wie Natrium oder deren Hydride wie Natriumhydrid einzusetzen. Bevorzugt sind Natriumhydrid, Kaliumcarbonat, Triethylamin, Pyridin und Kalium-tert.butylat.

Im allgemeinen setzt man die Base in einer Menge von 0,05 mol bis 10 mol, bevorzugt von 1 mol bis 2 mol, bezogen auf 1 mol der Verbindung der Formel (III), ein.

Das erfindungsgemäße Verfahren wird im allgemeinen in einem Temperaturbereich von -30°C bis +100°C, bevorzugt von -10°C bis +60°C, durchgeführt.

Das erfindungsgemäße Verfahren wird im allgemeinen bei Normaldruck durchgeführt. Es ist aber auch möglich, das Verfahren bei Überdruck oder bei Unterdruck durchzuführen (z.B. in einem Bereich von 0,5 bis 5 bar).

Als Basen eignen sich für die Hydrolyse die üblichen anorganischen Basen. Hierzu gehören bevorzugt Alkalihydroxide oder Erdalkalihydroxide wie beispielsweise Lithiumhydroxid, Natriumhydroxid, Kaliumhydroxid oder Bariumhydroxid, oder Alkalicarbonate wie Natrium- oder Kaliumcarbonat oder Natriumhydrogencarbonat, oder Alkalialkoholate wie Natriummethanolat, Natriumethanolat, Kaliummethanolat, Kaliumethanolat oder Kalium-tert.butanolat. Besonders bevorzugt wird Lithiumhydroxid, Natriumhydroxid oder Kaliumhydroxid eingesetzt.

Als Lösemittel eignen sich für die Verseifung Wasser oder die für eine Hydrolyse üblichen organischen Lösemittel. Hierzu gehören bevorzugt Alkohole wie Methanol, Ethanol, Propanol, Isopropanol oder Butanol, oder Ether wie Tetrahydrofuran oder Dioxan, oder Dimethylformamid, oder Dimethylsulfoxid. Besonders bevorzugt werden Alkohole wie Methanol, Ethanol, Propanol oder Isopropanol verwendet. Ebenso ist es möglich, Gemische der genannten Lösemittel einzusetzen.

Bevorzugt erfolgt die Hydrolyse mit Säuren wie beispielsweise Trifluoressigsäure, Essigsäure, Chlorwasserstoffsäure, Chlorwasserstoffsäure / Dioxan, Bromwasserstoffsäure, Methansulfonsäure, Schwefelsäure oder Perchlorsäure, besonders bevorzugt mit Trifluoressigsäure oder Chlorwasserstoffsäure / Dioxan.

Die Hydrolyse wird im allgemeinen in einem Temperaturbereich von 0°C bis +100°C, bevorzugt von +20°C bis +80°C, durchgeführt.

Im allgemeinen wird die Hydrolyse bei Normaldruck durchgeführt. Es ist aber auch möglich, bei Unterdruck oder bei Überdruck zu arbeiten (z.B. von 0,5 bis 5 bar).

Bei der Durchführung der Verseifung wird die Base im allgemeinen in einer Menge von 1 bis 3 mol, bevorzugt von 1 bis 1,5 mol, bezogen auf 1 mol des Esters, eingesetzt. Besonders bevorzugt verwendet man molare Mengen der Reaktanden.

Bei der Durchführung der Reaktion entstehen im ersten Schritt die Carboxylate der erfindungsgemäßen Verbindungen als Zwischenprodukte, die isoliert werden können. Die erfindungsgemäßen Säuren erhält man durch Behandeln der Carboxylate mit üblichen anorganischen Säuren. Hierzu gehören bevorzugt Säuren wie beispielsweise Chlorwasserstoffsäure, Bromwasserstoffsäure, Schwefelsäure, Phosphorsäure oder Trifluoressigsäure. Es hat sich bei der Herstellung der Carbonsäuren hierbei als vorteilhaft erwiesen, die basische Reaktionsmischung der Verseifung in einem zweiten Schritt ohne Isolierung der Carboxylate anzusäuern. Die Säuren können dann in üblicher Weise isoliert werden.

Die Amidierung und die Sulfoamidierung erfolgt im allgemeinen in einem der oben aufgeführten Lösemittel, vorzugsweise in Tetrahydrofuran oder Dichlormethan.

Die Amidierung oder Sulfoamidierung kann von den entsprechenden Säuren gegebenenfalls über die aktivierte Stufe der Säurehalogenide oder gemischten Anhydride, die aus den entsprechenden Säuren durch Umsetzung mit Thionylchlorid, Phosphortrichlorid, Phosphorpentachlorid, Phosphortribromid oder Oxalylchlorid oder Methansulfonsäurechlorid hergestellt werden können, verlaufen.

Die Amidierung oder Sulfoamidierung erfolgt im allgemeinen in einem Temperaturbereich von -50°C bis +80°C, vorzugsweise von -30°C bis +20°C, und Normaldruck.

Als Basen eignen sich dafür neben den oben aufgeführten Basen vorzugsweise Triethylamin und/oder Dimethylaminopyridin, DBU oder DABCO.

Die Base wird in einer Menge von 0,5 mol bis 10 mol, bevorzugt von 1 mol bis 5 mol, bezogen auf 1 mol der Verbindungen der allgemeinen Formel (V), eingesetzt.

Als säurebindende Mittel für die Amidierung oder Sulfoamidierung können Alkali- oder Erdalkalicarbonate wie Natriumcarbonat, Kaliumcarbonat, Alkali- oder Erdalkalihydroxide wie beispielsweise Natrium- oder Kaliumhydroxid, oder organische Basen wie Pyridin, Triethylamin, N-Methylpiperidin, oder bicyclische Amidine wie 1,5-Diazabicyclo[3.4.0]-nonene-5 (DBN) oder 1,5-Diazabicyclo[3.4.0]undecene-5 (DBU) eingesetzt werden. Bevorzugt ist Triethylamin.

Als Dehydratisierungsreagenzien eignen sich Carbodiimide wie beispielsweise Diisopropylcarbodiimid, Dicyclohexylcarbodiimid oder N-(3-Dimethylaminopropyl)-N'-ethylcarbodiimid-Hydrochlorid oder Carbonylverbindungen wie Carbonyldiimidazol oder 1,2-Oxazoliumverbindungen wie 2-Ethyl-5-phenyl-1,2-oxazolium-3-sulfonat oder Propanphosphonsäureanhydrid oder Isobutylchloroformat oder Benzotriazolyloxy-tris-(dimethylamino)phosphonium-hexafluorophosphat oder Phosphorsäurediphenylesteramid oder Methansulfonsäurechlorid, gegebenenfalls in Anwesenheit von Basen wie Triethylamin oder N-Ethylmorpholin oder N-Methylpiperidin oder Dicyclohexylcarbodiimid und N-Hydroxysuccinimid.

Die säurebindenden Mittel und Dehydratisierungsreagenzien werden im allgemeinen in einer Menge von 0,5 bis 3 mol, bevorzugt von 1 bis 1,5 mol, bezogen auf 1 mol der entsprechenden Carbonsäuren, eingesetzt.

Die oben aufgeführte Derivatisierung der Substituenten A, B, D und R¹ erfolgt im allgemeinen nach literaturbekannten Methoden, wobei beispielhaft die Reduktion von Aldehyden oder Alkoxycarbonylverbindungen zu Alkoholen (a), die Reduktion von Doppelbindungen (b) und die Alkylierung (c) mit folgendem erläutert werden sollen:
a) Die Reduktion von Carbonylverbindungen zu den entsprechenden Alkoholen erfolgt im allgemeinen mit Hydriden, wie Lithiumaluminiumhydrid oder Natriumborhydrid, bevorzugt im Fall der Alkoxycarbonylverbindungen mit Lithiumaluminiumhydrid und im Fall der Aldehyde bevorzugt mit Natriumborhydrid in inerten Lösemitteln wie Ethern, Kohlenwasserstoffen oder Alkoholen oder deren Gemischen, bevorzugt in Ethern wie beispielsweise Diethylether, Tetrahydrofuran oder Dioxan, oder Alkoholen wie Ethanol, im Fall der Aldehyde bevorzugt mit Natriumborhydrid in Ethanol, in einem Temperaturbereich von 0°C bis +150°C, bevorzugt von +20°C bis +100°C, bei Normaldruck.
   Die Reduktion einer Doppelbindung erfolgt im allgemeinen durch Hydrierung mit Wasserstoff in Anwesenheit eines Katalysators wie beispielsweise Platin oder Platinoxide, Rhodium, Ruthenium, Chlorotris(triphenylphosphin)rhodium, oder Palladium auf Tierkohle, bevorzugt mit Palladium auf Tierkohle in einem Temperaturbereich von 0°C bis +150°C, bevorzugt von +25°C bis +100°C.
b) Als Lösemittel für die Hydrierung eignen sich protische Lösemittel wie beispielsweise Methanol, Ethanol und/oder aprotische Lösemittel wie beispielsweise Tetrahydrofuran, Toluol, Dimethylformamid, Methylenchlorid, Dioxan oder Essigester.
   Die Hydrierung wird bei einem Druck von 1 bis 300 atm, vorzugsweise bei 1 bis 20 atm, durchgeführt.
c) Die Alkylierung erfolgt im allgemeinen in einem der oben aufgeführten Lösemitteln mit Alkylierungsmitteln wie beispielsweise (C₁-C₈)-Alkylhalogenide, Sulfonsäureester oder substituierte oder unsubstituierte (C₁-C₆)-Dialkyl- oder (C₁-C₁₀)-Diarylsulfate, vorzugsweise Methyljodid, p-Toluolsulfonsäureester oder Dimethylsulfat.

Die Verbindungen der allgemeinen Formel (II) sind teilweise neu und können beispielsweise hergestellt werden, indem man Verbindungen der allgemeinen Formel (VI) 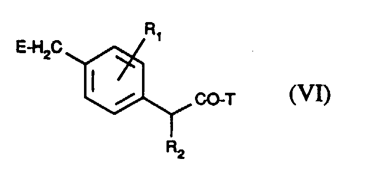 in welcher
- R¹ und R²: die oben angegebene Bedeutung haben,
- E: für eine typische Abgangsgruppe wie beispielsweise Chlor, Brom, Jod, Tosylat oder Mesylat, vorzugsweise für Brom steht,
und
- T: für geradkettiges oder verzweigtes (C₁-C₄)-Alkoxy steht,
mit Imidazolen der allgemeinen Formel (VII) 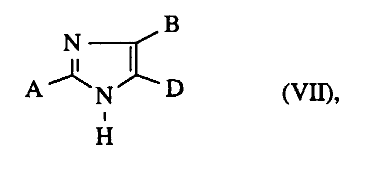 in welcher
A, B und D die oben angegebene Bedeutung haben,
in einem der oben aufgeführten Lösemitteln, vorzugsweise Dimethylformamid und in Anwesenheit einer der oben aufgeführten Basen, vorzugsweise Natriumhydrid oder Kaliumcarbonat, gegebenenfalls unter Schutzgasatmosphäre umsetzt,
und im Fall der Säuren die Ester wie oben beschrieben verseift.
Im allgemeinen setzt man die Base in einer Menge von 0,05 mol bis 10 mol, bevorzugt von 1 mol bis 2 mol bezogen auf 1 mol der Verbindung der Formel (VII) ein.

Das Verfahren wird im allgemeinen in einem Temperaturbereich von -30°C bis +100°C, bevorzugt von -10°C bis +10°C, durchgeführt.

Das Verfahren wird im allgemeinen bei Normaldruck durchgeführt. Es ist aber auch möglich, das Verfahren bei Überdruck oder bei Unterdruck durchzuführen (z.B. in einem Bereich von 0,5 bis 5 bar).

Die Verbindungen der allgemeinen Formeln (III), (IV), (V), (VI) und (VII) sind größtenteils bekannt oder nach üblichen Methoden herstellbar.

Die erfindungsgemäßen Verbindungen der allgemeinen Formel (I) zeigen ein nicht vorhersehbares, wertvolles pharmakologisches Wirkspektrum.

Die erfindungsgemäßen Verbindungen besitzen eine spezifische A II-antagonistische Wirkung, da sie kompetitiv die Bindung von Angiotensin II an die Rezeptoren hemmen. Sie unterdrücken die vasokonstriktorischen und Aldosteronsekretions-stimulierenden Effekte des Angiotensin II. Darüberhinaus inhibieren sie die Proliferation von glatten Muskelzellen.

Sie können deshalb in Arzneimitteln zur Behandlung der arteriellen Hypertonie und Atherosklerose eingesetzt werden. Darüberhinaus können sie zur Behandlung von coronaren Herzerkrankungen, Herzinsuffizienz, Störungen der Hirnleistung, ischämischen Gehirnerkrankungen, peripherer Durchblutungsstörungen, Funktionsstörungen der Niere und Nebenniere, bronchospastischen und vaskulär bedingten Erkrankungen der Atemwege, Natriumretention und Ödemen eingesetzt werden.

### Untersuchung auf die Hemmung der mit Agonisten induzierten Kontraktion

Kaninchen beiderlei Geschlechts werden durch Nackenschlag betäubt und entblutet, oder fallweise mit Nembutal (ca. 60 - 80 mg/kg i.v.) narkotisiert und durch Öffnung des Thorax getötet. Die Thoraxaorta wird entnommen, von anhaftendem Bindegewebe befreit, in 1,5 mm breite Ringsegmente geteilt und einzeln unter einer Anfangsbelastung von ca. 3,5 g in 10 ml Organbäder mit auf 37°C temperierter, Carbogen-begaster Krebs-Henseleit-Nährlösung folgender Zusammensetzung: 119 mmol/l NaCl; 2,5 mmol/l CaCl₂ x 2 H₂O; 1,2 mmol/l KH₂PO₄; 10 mmol/l Glucose; 4,8 mmol/l KCl; 1,4 mmol/l MgSO₄ x 7 H₂O und 25 mmol/l NaHCO₃ verbracht.

Die Kontraktionen werden isometrisch durch Statham UC2-Zellen über Brückenverstärker (ifd Mülheim bzw. DSM Aalen) erfaßt und mittels A/D-Wandler (System 570, Keithley München) digitalisiert sowie ausgewertet. Die Durchführung von Agonistdosiswirkungskurven (DWK) erfolgt stündlich. Mit jeder DWK werden 3 bzw. 4 Einzelkonzentrationen im 4 min-Abstand in die Bäder appliziert. Nach Ende der DWK und darauffolgender Auswaschzyklen (16 mal jeweils ca. 5 sec/min mit der o.a. Nährlösung) schließt sich eine 28-minütige Ruhe- bzw. Inkubationsphase an, innerhalb derer die Kontraktionen in der Regel wieder den Ausgangswert erreichen.

Die Höhe der im Normalfall 3. DWK wird als Bezugsgröße für die Bewertung der in weiteren Durchgängen zu untersuchenden Testsubstanz verwendet, die bei den nachfolgenden DWK's in jeweils steigender Dosierung mit Beginn der Inkubationszeit in die Bäder appliziert wird. Jeder Aortenring wird dabei ganztägig mit immer dem gleichen Agonisten stimuliert.

### Agonisten und ihre Standardkonzentrationen (Applikationsvolumen pro Einzelgabe = 100 µl):

| | | |
|---|---|---|
| KCl | 22,7;32,7;42,7;52,7 | mmol/l |
| L-Noradrenalin | 3x10⁻⁹;3x10⁻⁸;3x10⁻⁷;3x10⁻⁶ | g/ml |
| Serotonin | 10⁻⁸;10⁻⁷;10⁻⁶;10⁻⁵ | g/ml |
| B-HT 920 | 10⁻⁷;10⁻⁶;10⁻⁵ | g/ml |
| Methoxamin | 10⁻⁷;10⁻⁶;10⁻⁵ | g/ml |
| Angiotensin II | 3x10⁻⁹;10⁻⁸;3x10⁻⁸;10⁻⁷ | g/ml |

Für die Berechnung der IC₅₀ (Konzentration bei der die zu untersuchende Substanz eine 50%ige Hemmung verursacht) wird der Effekt jeweils an der 3. = submaximalen Agonistkonzentration zugrundegelegt.

Die erfindungsgemäßen Verbindungen hemmen die durch Angiotensin II induzierte Kontraktion der isolierten Kaninchenaorta dosenabhängig. Die durch Kalium-Depolarisation oder andere Agonisten induzierte Kontraktion wurde nicht oder in hohen Konzentrationen nur schwach gehemmt.

### Blutdruckmessungen an der Angiotensin II-infundierten Ratte

Männliche Wistar Ratten (Moellegaard, Kopenhagen, Dänemark) mit einem Körpergewicht von 300 - 350 g, werden mit Thiopental (100 mg/kg i.p.) anästhesiert. Nach Tracheotomie wird in die Femoralarterie ein Katheter zur Blutdruckmessung und in die Femoralvenen ein Katheter für die Angiotensin II-Infusion und ein Katheter für die Substanzverabreichung eingeführt. Nach Gabe des Ganglienblockers Pentolinium (5 mg/kg i.v.) wird die Angiotensin II-Infüsion (0,3 µg/kg/min) gestartet. Sobald die Blutdruckwerte ein stabiles Plateau erreicht haben, werden die Testsubstanzen entweder intravenös oder als Suspension bzw. Lösung in 0,5% Tylose oral verabreicht.

### Bestimmung der antihypertensiven Wirksamkeit bei wachen hypertensiven Ratten

Die orale antihypertensive Wirksamkeit der erfindungsgemäßen Verbindungen wurde an wachen Ratten mit chirurgisch induzierter unilateraler Nierenarterienstenose geprüft Dazu wurde die rechte Nierenarterie mit einem Silberclip von 0,18 mm lichter Weite eingeengt. Bei dieser Hypertonieform ist die Plasmareninaktivität in den ersten sechs Wochen nach dem Eingriff erhöht.

Der arterielle Blutdruck diese Tiere wurde in definierten Zeitabständen nach Substanzgabe mit der "Schwanzmanschette" unblutig gemessen. Die zu prüfenden Substanzen wurden aufgeschwemmt in einer Tylosesuspension intragastral ("oral") per Schlundsonde in verschiedenen Dosen appliziert. Die erfindungsgemäßen Verbindungen senken den arteriellen Blutdruck der Hochdruckratten in klinisch relevanter Dosierung.

Außerdem hemmen die erfindungsgemäßen Verbindungen die spezifische Bindung von radioaktivem Angiotensin II konzentrationsabhängig.

### Interaktion der erfindungsgemäßen Verbindungen mit dem Angiotensin II-Rezeptor an Membranfraktionen der Nebennierenrinde (Rind)

Nebennierenrinden vom Rind (NNR), die frisch entnommen und sorgfältig von Mark von Kapsel befreit sind, werden in Sucrose-Lösung (0,32 M) mit Hilfe eines Ultra-Turrax (Janke & Kunkel, Staufen i.B.) zu grobem Membran-Homogenat zerkleinert und in zwei Zentrifugationsschritten zu Membranfraktionen partiell aufgereinigt.
Die Untersuchungen zur Rezeptor-Bindung werden an partiell gereinigten Membranfraktionen boviner NNR mit radioaktivem Angiotensin II in einem Assay-Volumen von 0,25 ml durchgeführt, das im einzelnen die partiell gereinigten Membranen (50 - 80 µg), ³H-Angiotensin II (3-5 nM), Test-Pufferlösung (50 mM Tris, pH 7,2), 5 mM MgCl₂ sowie die zu untersuchenden Substanzen enthält. Nach einer Inkubationszeit von 60 min bei Raumtemperatur wird die nicht gebundene Radioaktivität der Proben mittels angefeuchteter Glasfaserfilter (Whatman GF/C) separiert und die gebundene Radioaktivität nach Waschung des Proteins mit eiskalter Pufferlösung (50 mM Tris/HCl, pH 7,4, 5% PEG 6000) in einem Szintillationscocktail spektrophotometrisch gemessen. Die Analyse der Rohdaten erfolgte mit Computer-Programmen zu Kᵢ- bzw. IC₅₀-Werten (Kᵢ: für die verwendete Radioaktivität korrigierte IC₅₀-Werte; IC₅₀-Werte: Konzentration bei der die zu untersuchende Substanz eine 50%ige Hemmung der spezifischen Bindung des Radioliganden bewirkt).

**Tabelle A:**

| Bsp.-Nr. | IC₅₀ [nM] |
|---|---|
| 7 | 580 |
| 12 | 290 |

### Untersuchung zur Inhibition der Proliferation glatter Muskelzellen durch die erfindungsgemäßen Verbindungen

Zur Feststellung der antiproliferativen Wirkung der Verbindungen werden glatte Muskelzellen verwendet, die aus den Aorten von Ratten durch die Media-Explantat-Technik gewonnen werden [R. Ross, J. Cell. Biol. 50, 172, 1971]. Die Zellen werden in geeigneten Kulturschalen, in der Regel 96-Loch-Platten, ausgesät und für 2 - 3 Tage in Medium 199 mit 7,5% FCS und 7,5% NCS, 2 mM L-Glutamin und 15 mM HEPES, pH 7,4 in 5% CO₂ bei 37°C kultiviert. Danach werden die Zellen durch Serumentzug für 2 - 3 Tage synchronisiert und sodann mit Serum oder anderen Faktoren zum Wachstum angeregt. Gleichzeitig werden Testverbindungen zugesetzt Nach 16 - 20 Stunden wird 1 µCi ³H-Thymidin zugefügt und nach weiteren 4 Stunden der Einbau dieser Substanz in die TCA-präzipitierbare DNA der Zellen bestimmt. Zur Bestimmung der IC₅₀-Werte wird die Wirkstoffkonzentration errechnet, die bei sequentieller Verdünnung des Wirkstoffes halbmaximale Hemmung der durch 10% FCS hervorgerufene Thymidininkorporation bewirkt.

**Tabelle B:**

| Bsp.-Nr. | IC₅₀ [nM] |
|---|---|
| 9 | 860 |
| 14 | 450 |

Die neuen Wirkstoffe können in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nichttoxischer, pharmazeutisch geeigneter Trägerstoffe oder Lösemittel. Hierbei soll die therapeutisch wirksame Verbindung jeweils in einer Konzentration von etwa 0,5 bis 90-Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösemitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösemittel als Hilfslösemittel verwendet werden können.

Die Applikation erfolgt in üblicher Weise, vorzugsweise oral oder parenteral, insbesondere perlingual oder intravenös.

Für den Fall der parenteralen Anwendung können Lösungen des Wirkstoffs unter Verwendung geeigneter flüssiger Trägermaterialien eingesetzt werden.

Im allgemeinen hat es sich als vorteilhaft erwiesen, bei intravenöser Applikation Mengen von etwa 0,001 bis 1 mg/kg, vorzugsweise etwa 0,01 bis 0,5 mg/kg Körpergewicht zur Erzielung wirksamer Ergebnisse zu verabreichen, und bei oraler Applikation beträgt die Dosierung etwa 0,01 bis 20 mg/kg, vorzugsweise 0,1 bis 10 mg/kg Körpergewicht.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehreren Einzelgaben über den Tag zu verteilen.

### Laufmittel

(A) Dichlormethan / Methanol / Ammoniak = 9:1:0,1
(B) Essigester / Petrolether = 7:3
(C) Essigester / Petrolether = 3:7
(D) Dichlormethan / Methanol = 9:1
(E) Dichlormethan / Methanol / Essigsäure = 9:1:0,1

### Ausgangsverbindungen

### Beispiel I

4-Methylphenylessigsäure-tert.butylester 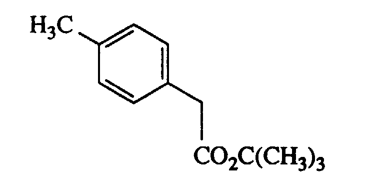 450 g (3 mol) 4-Methylphenylessigsäure, 1,13 l (12 mol) tert.Butanol und 90 g (0,74 mol) Dimethylaminopyridin werden in 2 l Dichlormethan gelöst. Nach Zugabe von 680 g (3,3 mol) Dicyclohexylcarbodiimid, gelöst in 400 ml Dichlormethan, wird 20 h bei 25°C gerührt, der ausgefallene Harnstoff abgesaugt, mit 200 ml Dichlormethan gewaschen und die organische Phase je zweimal mit 500 ml 2 N Salzsäure und Wasser gewaschen. Die organische Phase wird eingeengt und destilliert.
Ausbeute: 408 g (66% der Theorie)
Siedepunkt: 73- 78°C / 0,2 mm

### Beispiel II

2-Cyclopentyl-2-(4-methylphenyl)essigsäure-tert.butylester 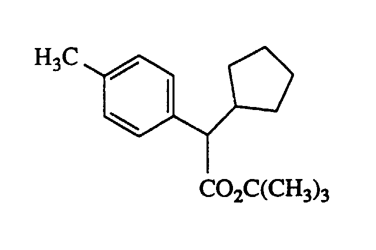 33,5 g (0,3 mol) Kalium-tert.butylat werden in 100 ml DMF unter Feuchtigkeitsausschluß bei 0°C vorgelegt, und 51,6 g (0,25 mol) 4-Methylphenylessigsäure-tert.butylester in 250 ml DMF zugetropft. Es wird 30 min bei 0°C gerührt und 32,2 ml (0,3 mol) Cyclopentylbromid in 150 ml DMF bei 5-15°C zugetropft und 20 h bei 25°C gerührt. Nach Einengen wird der Rückstand zwischen Wasser/Diethylether verteilt, die Etherphase über Natriumsulfat getrocknet und eingeengt. Das Produkt kristallisiert aus.
Ausbeute: 67 g (97,5% der Theorie)
Festpunkt: 51 - 53°C

### Beispiel III

2-(4-Brommethyl-phenyl)-2-cyclopentyl-essigsäure-tert.butylester 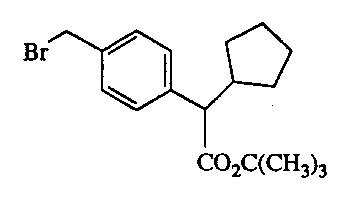 27,4 g (0,1 mol) 2-Cyclopentyl-2-(4-methylphenyl)-essigsäure-tert.butylester werden in 200 ml Tetrachlorkohlenstoff gelöst und zum Sieden erhitzt. Nach Zugabe von 0,82 g Azobisisobutyronitril werden 18,7 g (0,105 mol) N-Bromsuccinimid portionsweise zugegeben und anschließend 1 h refluxiert, auf 0°C abgekühlt und vom Succinimid abfiltriert. Nach Einengen des Filtrates fällt das Produkt aus. Es wird mit Petrolether (40/60) gewaschen und getrocknet.
Ausbeute: 20 g (57% der Theorie)
Festpunkt: 73 - 76°C

### Beispiel IV

2-[4-(2-Butyl-4-chlor-5-formyl-imidazol-1-yl-methyl)phenyl]-2-cyclopentylessigsäure-tert.butylester 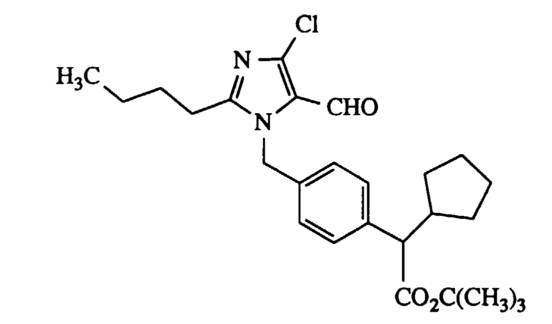 Unter Schutzgas werden 1,6 g (0,053 mol) Natriumhydrid (80%ig) in 50 ml DMF suspendiert, 10 g (0,053 mol) 2-Butyl-5-formyl-4-chlorimidazol (Herstellung nach EP 324 377) in 100 ml DMF bei 0°C zugetropft, anschließend bei 0°C 15 min gerührt und 18,9 g (0,053 mol) 2-(4-Brommethylphenyl)-2-cyclopentylessigsäure-tert.butylester in 100 ml DMF zugetropft. Es wird 2 h bei 0°C nachgerührt, das Lösemittel abgedampft, der Rückstand in Diethylether aufgenommen, abfiltriert und nach Einengen über Kieselgel 60 mit Dichlormethan chromatographiert.
Ausbeute: 16,2 g (66,7% der Theorie)
Festpunkt: 101-102°C

### Beispiel V

2-[4-(2-Butyl-4-chlor-5-formyl-imidazol-1-yl-methyl)phenyl]-2-cyclopentylessigsäure 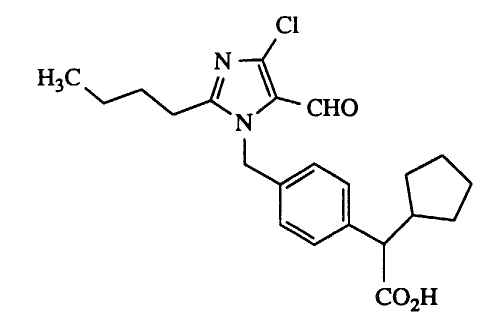 2,3 g (5 mmol) der Verbindung aus Beispiel IV werden in 5 ml Dichlormethan und 5 ml Trifluoressigsäure 5 h bei 25°C gerührt. Nach Einengen wird das Rohprodukt über Kieselgel 60 mit Dichlormethan/Methanol (100:5) chromatographiert.
Ausbeute: 1,8 g (87,6% der Theorie)
Festpunkt: 95-98°C

### Herstellungsbeispiele

### Beispiel 1 und Beispiel 2

2-[4-(2-Butyl-4-chlor-5-formyl-imidazol-1-yl-methyl)phenyl]-2-cyclopentylessigsäure-L-(prolin-tert.butylester)amid 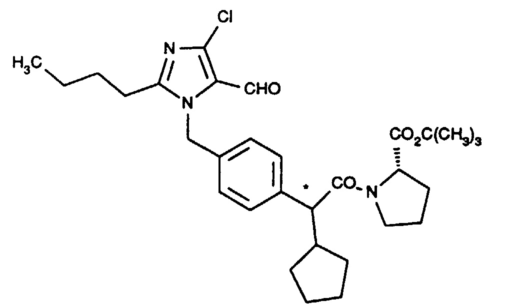 1,0 g (2,5 mmol) der Verbindung aus Beispiel V werden in 30 ml Dichlormethan bei Raumtemperatur mit 0,4 g (2,7 mmol) 1-Hydroxy-1H-benzotriazol versetzt, auf 0°C gekühlt, und anschließend werden 0,51 g (5 mmol) Triethylamin sowie eine Lösung von 0,56 g (2,7 mmol) Dicyclohexylcarbodiimid in 10 ml Dichlormethan zugetropft. Nach 30 Minuten wird bei 0°C eine Lösung von 0,51 g (3,0 mmol) L-Prolin-tert.butylester in 10 ml Dichlormethan zugegeben und über Nacht bei Raumtemperatur gerührt. Zur Aufarbeitung wird mit Wasser und Dichlormethan versetzt, geschüttelt, getrennt, die wäßrige Phase noch zweimal mit Dichlormethan extrahiert, und die vereinigten organischen Phasen werden über Natriumsulfat getrocknet, filtriert, eingeengt und über Kieselgel 60 (Essigester / Petrolether = 1:1) chromatographiert.
Gesamtausbeute: 1,14 g (2,0 mmol)
dia A und dia B: 82% der Theorie
R_{f} = 0,84 (dia A) (Beispiel 1) (Essigester/Petrolether = 7:3)
0,79 (dia B) (Beispiel 2) (Essigester/Petrolether =7:3)

In Analogie zur Vorschrift der Beispiele 1 und 2 wird die in Tabelle 1 aufgeführte Verbindung hergestellt:

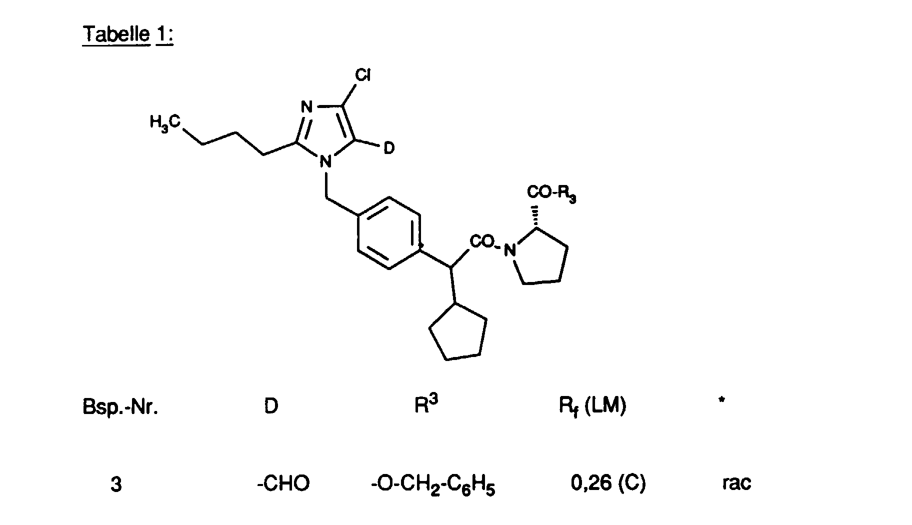

### Beispiel 4

2-[4-(2-Butyl-4-chlor-5-formyl-imidazol-1-yl-methyl)phenyl]-2-cyclopentylessigsäure-L-prolinamid 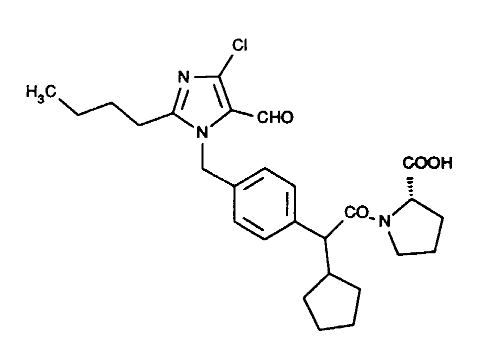 In Analogie zur Vorschrift des Beispiels V wird die Titelverbindung durch Esterspaltung von 0,6 g (1,1 mmol) der Verbindung aus Beispiel 1 hergestellt.
Ausbeute: 0,48 g (1 mmol) 98% d.Th.
R_{f} = 0,53 (Dichlormethan / Methanol 9:1)

### Beispiel 5

2-[4-(2-Butyl-4-chlor-5-formyl-imidazol-1-yl-methyl)phenyl]-2-cyclopentylessigsäure-(L-prolinamido)amid 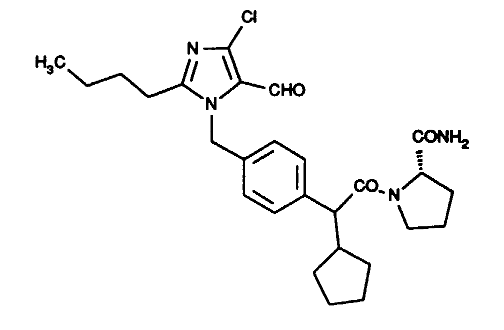 Die Titelverbindung wird in Analogie zur Vorschrift der Beispiele 1 und 2 aus 1,0 g (2,5 mmol) der Verbindung aus Beispiel V und 0,34 g (3,0 mmol) L-Prolinamid hergestellt.
Ausbeute: 0,91 g (1,8 mmol) 73% d.Th.
R_{f} = 0,65 (Dichlormethan / Methanol / Ammoniak = 9:1:0,1)

### Beispiel 6

2-[4-(2-Butyl-4-chlor-5-carboxy-imidazol-1-yl-methyl)phenyl]-2-cyclopentylessigsäure-L-prolinamid 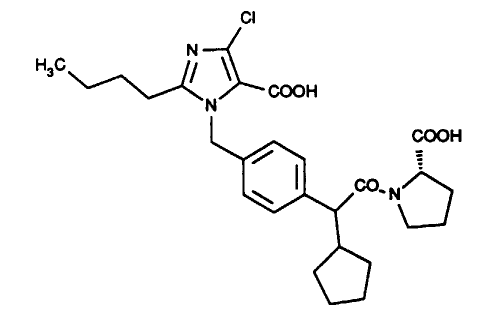 0,2 g (0,4 mmol) der Verbindung aus Beispiel 4 werden in einem Gemisch aus 3 ml tert.Butanol und 1,6 ml 1,25 M Natriumdihydrogenphosphatlösung (pH 7) bei Raumtemperatur mit 2,4 ml 1 M Kaliumpermanganatlösung versetzt und 10 Minuten gerührt. Nach Zugabe von 5 ml gesättigter Natriumsulfitlösung (Eiskühlung) wird mit 1 N HCl angesäuert und dreimal mit Essigester extrahiert. Die vereinigten organischen Phasen werden über Natriumsulfat getrocknet, filtriert, eingeengt und über Kieselgel 60 (Dichlormethan / Methanol = 9:1) chromatographiert.
Ausbeute: 0,06 g (0,12 mmol) 30,6% d.Th.
R_{f} = 0,35 (Dichlormethan / Methanol / Essigsäure = 9:1:0,1)

### Beispiel 7

2-[4-(2-Butyl-4-chlor-5-carboxy-imidazol-1-yl-methyl)phenyl]-2-cyclopentylessigsäure-(L-prolinamido)amid 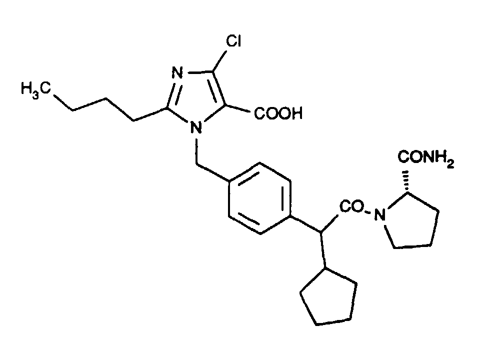 In Analogie zur Vorschrift des Beispiels 6 wird die Titelverbindung aus 0,3 g (0,6 mmol) der Verbindung aus Beispiel 5 hergestellt.
Ausbeute: 0,29 g (0,57 mmol) 94% d.Th.
R_{f} = 0,31 (Dichlormethan / Methanol / Eisessig = 9:1:0,1)

### Beispiel 8

2-[4-(2-Butyl-4-chlor-5-formyl-imidazol-1-yl-methyl)phenyl]-2-cyclopentylessigsäure-(L-prolin-phenylglycinolamido)amid 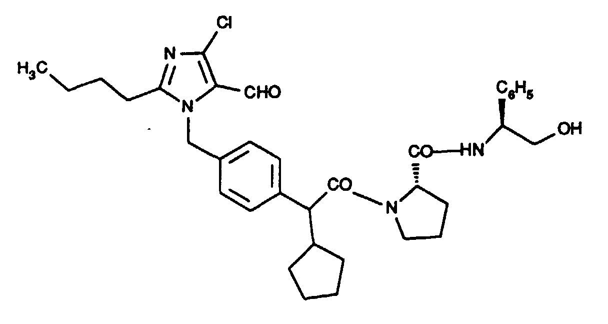 0,11 g (0,22 mmol) der Verbindung aus Beispiel 4 werden in 5 ml Tetrahydrofuran bei Raumtemperatur mit 0,05 ml (0,44 mmol) Triethylamin versetzt, und unter Rühren auf -30°C gekühlt. Man gibt 0,03 g (0,24 mmol) Methansulfonsäurechlorid zu und läßt nach 90 Minuten Rühren eine Lösung von 0,03 g (0,24 mmol) L-Phenylglycinol in 5 ml Tetrahydrofuran zutropfen, rührt noch 1 Stunde bei -30°C und läßt über Nacht auf Raumtemperatur erwärmen. Zur Aufarbeitung wird mit 5 ml 1 N Essigsäure versetzt, Essigester und Wasser zugegeben, geschüttelt und die wäßrige Phase noch zweimal mit Essigester extrahiert. Die vereinigten organischen Phasen werden über Natriumsulfat getrocknet, filtriert, eingeengt und über Kieselgel 60 (Dichlormethan / Methanol = 98:2) chromatographiert.
Ausbeute: 40,3 mg (0,065 mmol) 29,5% d.Th.
R_{f} = 0,21 (Essigester / Petrolether = 7:3)

### Beispiel 9

2-[4-(2-Butyl-4-chlor-5-hydroxymethyl-imidazol-1-yl-methyl)phenyl]-2-cyclopentylessigsäure-(L-prolin-phenylglycinolamido)amid 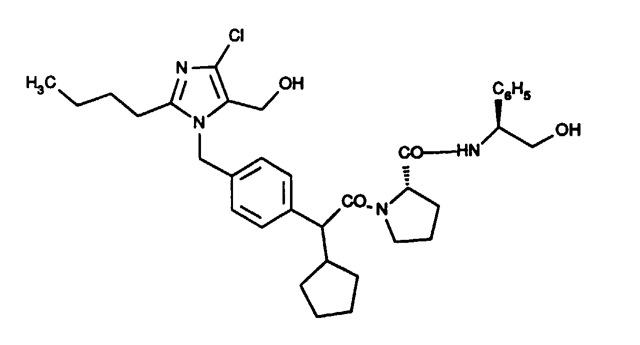 0,04 g (0,06 mmol) der Verbindung aus Beispiel 7 werden in 2 ml Ethanol gelöst, mit 2 mg (0,06 mmol) Natriumborhydrid versetzt und 2 Stunden bei Raumtemperatur gerührt. Zur Aufarbeitung wird mit Wasser versetzt, mit 1 N Essigsäure angesäuert, dreimal mit Essigester extrahiert, und die vereinigten organischen Phasen werden über Magnesiumsulfat getrocknet, filtriert und eingeengt.
Ausbeute: 0,03 g (0,05 mmol) 89% d.Th.
R_{f} = 0,52 (Dichlormethan: Methanol = 9:1)

In Analogie zu den Vorschriften der Beispiele 6, 8 und 9 werden die in Tabelle 2 aufgeführten Verbindungen hergestellt:

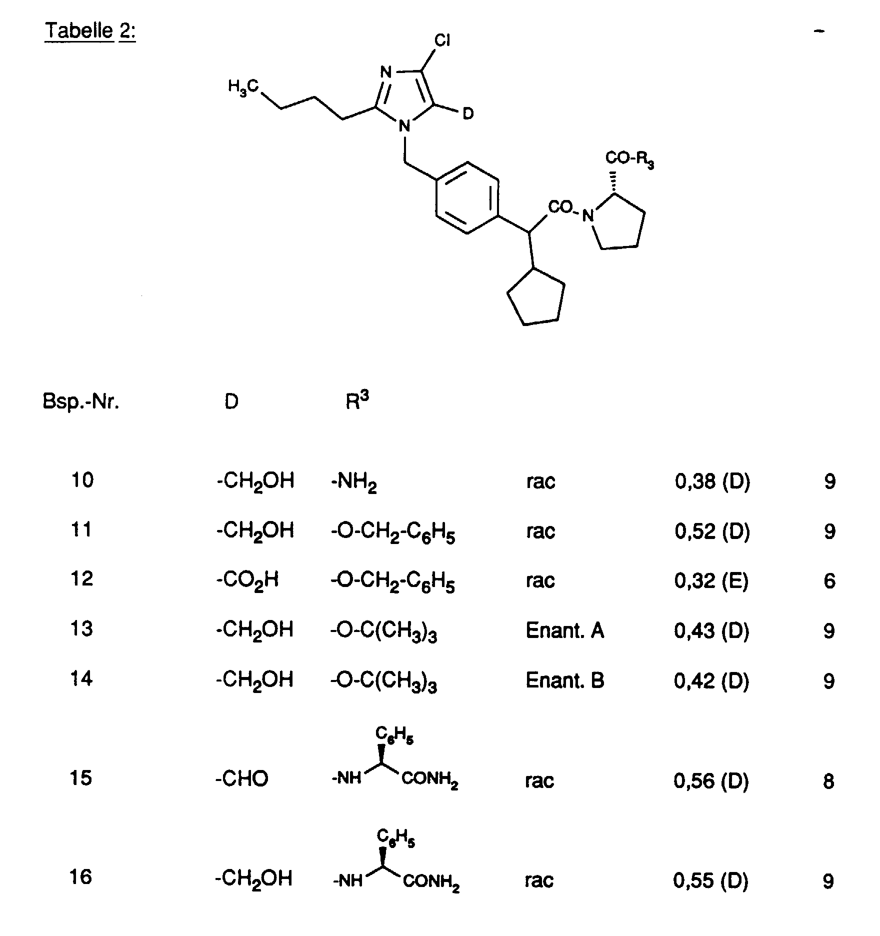

## Patentansprüche

1. Imidazolyl-substituierte Phenylessigsäureprolinamide der allgemeinen Formel 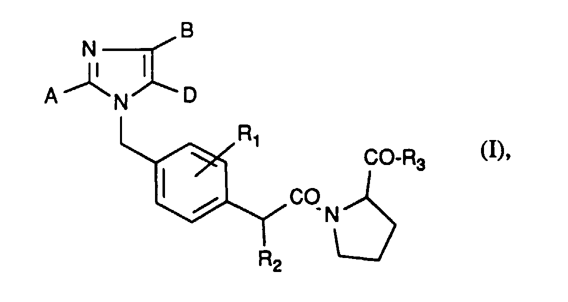 in welcher
A für geradkettiges oder verzweigtes Alkyl oder Alkenyl mit jeweils bis zu 8 Kohlenstoffatomen steht, oder für Cycloalkyl mit 3 bis 8 Kohlenstoffatomen steht,
B für Wasserstoff, Halogen oder für Perfluoralkyl mit bis zu 5 Kohlenstoffatomen steht,
D für eine Gruppe der Formel-CH₂-OR⁴ oder -CO-R⁵ steht,
worin
R⁴ Wasserstoff oder geradkettiges oder verzweigtes Alkyl mit bis zu 8 Kohlenstoffatomen bedeutet,
R⁵ Wasserstoff, Hydroxy oder geradkettiges oder verzweigtes Alkoxy mit bis zu 8 Kohlenstoffatomen bedeutet,
R¹ für Wasserstoff, Halogen, Nitro, Hydroxy, Trifluormethyl, Trifluormethoxy, geradkettiges oder verzweigtes Alkyl, Alkoxy oder Alkoxycarbonyl carbonyl mit jeweils bis zu 6 Kohlenstoffatomen, Cyano oder steht,
R² für Wasserstoff, geradkettiges oder verzweigtes Alkyl mit bis zu 8 Kohlenstoffatomen oder Cycloalkyl mit 3 bis 8 Kohlenstoffatomen steht,
R³ für Hydroxy, Benzyloxy oder für geradkettiges oder verzweigtes Alkoxy mit bis zu 8 Kohlenstoffatomen steht, oder
für eine Gruppe der Formel
-NR⁶R⁷ oder 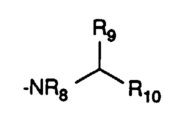 steht,
worin
R⁶, R⁷ und R⁸ gleich oder verschieden sind und Wasserstoff oder geradkettiges oder verzweigtes Alkyl mit bis zu 8 Kohlenstoffatomen bedeuten,
oder
R⁶ die oben angegebene Bedeutung hat
und
R⁷ einen Rest der Formel -SO₂R¹¹ bedeutet,
worin
R¹¹ geradkettiges oder verzweigtes Alkyl mit bis zu 4 Kohlenstoffatomen bedeutet, das gegebenenfalls durch Phenyl oder Cycloalkyl mit 3 bis 6 Kohlenstoffatomen substituiert ist, oder
Phenyl bedeutet, das gegebenenfalls durch geradkettiges oder verzweigtes Alkyl mit bis zu 4 Kohlenstoffatomen substituiert ist,
R⁹ Phenyl bedeutet, das gegebenenfalls bis zu 2-fach gleich oder verschieden durch Halogen, Nitro, Hydroxy, Trifluormethyl, Trifluormethoxy, geradkettiges oder verzweigtes Alkyl, Alkoxy oder Alkoxycarbonyl mit jeweils bis zu 6 Kohlenstoffatomen, Cyano, Carboxy, Cycloalkyl mit 3 bis 6 Kohlenstoffatomen oder Phenyl substituiert ist,
R¹⁰ Carboxy oder geradkettiges oder verzweigtes Alkoxycarbonyl mit bis zu 8 Kohlenstoffatomen bedeutet, oder
einen Rest der Formel -CH₂OR¹², -CO-NR¹³R¹⁴ oder -CH₂NR¹³R¹⁴ bedeutet,
worin
R¹² Wasserstoff, Benzyl oder geradkettiges oder verzweigtes Alkyl mit bis zu 8 Kohlenstoffatomen bedeutet,
R¹³ und R¹⁴ gleich oder verschieden sind und Wasserstoff, Phenyl oder geradkettiges oder verzweigtes Alkyl mit bis zu 6 Kohlenstoffatomen bedeuten,
gegebenenfalls in einer isomeren Form und deren Salze.

2. Imidazolyl-substituierte Phenylessigsäureprolinamide der Formel (I) nach Anspruch 1, wobei
A für geradkettiges oder verzweigtes Alkyl oder Alkenyl mit jeweils bis zu 6 Kohlenstoffatomen steht, oder
für Cyclopropyl, Cyclobutyl, Cyclopentyl, Cyclohexyl oder Cycloheptyl steht,
B für Wasserstoff, Fluor, Chlor, Brom oder für Perfluoralkyl mit bis zu 4 Kohlenstoffatomen steht,
D für eine Gruppe der Formel -CH₂OR⁴ oder -CO-R⁵ steht,
worin
R⁴ Wasserstoff oder geradkettiges oder verzweigtes Alkyl mit bis zu 6 Kohlenstoffatomen bedeutet,
R⁵ Wasserstoff, Hydroxy oder geradkettiges oder verzweigtes Alkoxy mit bis zu 6 Kohlenstoffatomen bedeutet,
R¹ für Wasserstoff, Fluor, Chlor, Brom, Trifluormethyl, Cyano oder geradkettiges oder verzweigtes Alkyl, Alkoxy oder Alkoxycarbonyl mit jeweils bis zu 5 Kohlenstoffatomen steht,
R² für Wasserstoff, geradkettiges oder verzweigtes Alkyl mit bis zu 6 Kohlenstoffatomen, Cyclopentyl, Cyclohexyl oder Cycloheptyl steht,
R³ für Hydroxy, Benzyloxy oder für geradkettiges oder verzweigtes Alkoxy mit bis zu 6 Kohlenstoffatomen steht, oder
für eine Gruppe der Formel
-NR⁶R⁷ oder 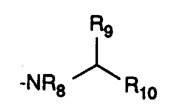 steht,
worin
R⁶, R⁷ und R⁸ gleich oder verschieden sind und Wasserstoff oder geradkettiges oder verzweigtes Alkyl mit bis zu 6 Kohlenstoffatomen bedeuten,
oder
R⁶ die oben angegebene Bedeutung hat
und
R⁷ einen Rest der Formel -SO₂R¹¹ bedeutet,
worin
R¹¹ geradkettiges oder verzweigtes Alkyl mit bis zu 3 Kohlenstoffatomen, Benzyl oder p-Tolyl bedeutet,
R⁹ Phenyl bedeutet, das gegebenenfalls durch Fluor, Chlor, Brom, Trifluormethyl, Carboxy oder geradkettiges oder verzweigtes Alkyl, Alkoxy oder Alkoxycarbonyl mit jeweils bis zu 4 Kohlenstoffatomen substituiert ist,
R¹⁰ Carboxy oder geradkettiges oder verzweigtes Alkoxycarbonyl mit bis zu 6 Kohlenstoffatomen bedeutet, oder
einen Rest der Formel -CH₂-OR¹², -CO-NR¹³R¹⁴ oder -CH₂NR¹³R¹⁴ bedeutet,
worin
R¹² Wasserstoff, Benzyl oder geradkettiges oder verzweigtes Alkyl mit bis zu 6 Kohlenstoffatomen bedeutet,
R¹³ und R¹⁴ gleich oder verschieden sind und Wasserstoff, Phenyl oder geradkettiges oder verzweigtes Alkyl mit bis zu 4 Kohlenstoffatomen bedeuten,
gegebenenfalls in einer isomeren Form und deren Salze.

3. Imidazolyl-substituierte Phenylessigsäureprolinamide der Formel (I) nach Anspruch 1, wobei
A für geradkettiges oder verzweigtes Alkyl oder Alkenyl mit jeweils bis zu 4 Kohlenstoffatomen steht, oder für Cyclopropyl, Cyclopentyl oder Cyclohexyl steht,
B für Wasserstoff, Fluor, Chlor oder für Perfluoralkyl mit bis zu 2 Kohlenstoffatomen steht,
D für eine Gruppe der Formel -CH₂OR⁴ oder -CO-R⁵ steht,
worin
R⁴ Wasserstoff oder geradkettiges oder verzweigtes Alkyl mit bis zu 4 Kohlenstoffatomen bedeutet,
R⁵ Wasserstoff, Hydroxy oder geradkettiges oder verzweigtes Alkoxy mit bis zu 4 Kohlenstoffatomen bedeutet,
R¹ für Wasserstoff, Fluor, Chlor, Brom, Trifluormethyl, Trifluormethoxy, Methyl oder Isobutyl steht,
R² für Wasserstoff, geradkettiges oder verzweigtes Alkyl mit bis zu 4 Kohlenstoffatomen, Cyclopentyl, Cyclohexyl oder Cycloheptyl steht,
R³ für Hydroxy, Benzyloxy oder für geradkettiges oder verzweigtes Alkoxy mit bis zu 4 Kohlenstoffatomen steht, oder
für eine Gruppe der Formel
-NR⁶R⁷ oder 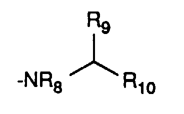 steht,
worin
R⁶, R⁷ und R⁸ gleich oder verschieden sind und Wasserstoff oder geradkettiges oder verzweigtes Alkyl mit bis zu 4 Kohlenstoffatomen bedeuten,
oder
R⁶ die oben angegebene Bedeutung hat
und
R⁷ einen Rest der Formel -SO₂R¹¹ bedeutet,
worin
R¹¹ Methyl, Benzyl oder p-Tolyl bedeutet,
R⁹ Phenyl bedeutet, das gegebenenfalls durch Fluor, Chlor, Brom, Trifluormethyl, Trifluormethoxy oder Methyl substituiert ist,
R¹⁰ Carboxy oder geradkettiges oder verzweigtes Alkoxycarbonyl mit bis zu 4 Kohlenstoffatomen bedeutet, oder
einen Rest der Formel -CH₂-OR¹², -CO-NR¹³R¹⁴ oder -CH₂NR¹³R¹⁴ bedeutet,
worin
R¹² Wasserstoff, Benzyl oder geradkettiges oder verzweigtes Alkyl mit bis zu 4 Kohlenstoffatomen bedeutet,
R¹³ und R¹⁴ gleich oder verschieden sind und Wasserstoff oder geradkettiges oder verzweigtes Alkyl mit bis zu 3 Kohlenstoffatomen bedeuten,
gegebenenfalls in einer isomeren Form und deren Salze.

4. Imidazolyl-substituierte Phenylessigsäureprolinamide nach Anspruch 1, wobei
R² für Cyclopentyl oder Cycloheptyl steht.

5. Imidazolyl-substituierte Phenylessigsäureprolinamide nach Ansprüchen 1 bis 4 zur therapeutischen Anwendung.

6. Verfahren zur Herstellung von Imidazolyl-substituierten Phenylessigsäureprolinamiden nach Ansprüchen 1 bis 4, dadurch gekennzeichnet, daß man
Verbindungen der allgemeinen Formel (II) 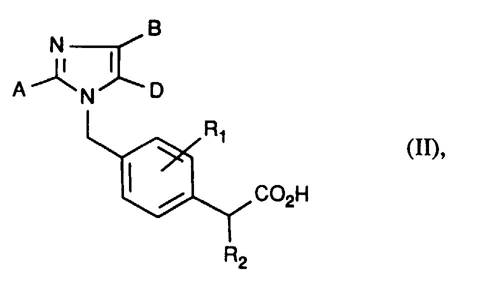 in welcher
A, B, D, R¹ und R² die oben angegebene Bedeutung haben,
mit Prolinderivaten der allgemeinen Formel (III) 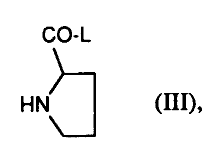 in welcher
L für geradkettiges oder verzweiges (C₁-C₄)-Alkoxy oder Benzyloxy steht,
in inerten Lösemitteln, in Anwesenheit einer Base und/oder eines Dehydratisierungsmittels umsetzt,
und im Fall der Prolinsäuren (R³ = OH) die Ester verseift,
und im Fall der Prolinamide (R⁶/R⁷ = H oder Alkyl) ausgehend von den Säuren, gegebenenfalls nach vorgeschalteter Aktivierung, wie oben beschrieben, mit Aminen der allgemeinen Formel (IV)
HNR⁶R⁷ (IV),
in welcher
R⁶ und R⁷ die oben angegebene Bedeutung haben,
umsetzt,
und im Fall der Prolin-Phenylglycinamide (-NR⁸-CHR⁹-R¹⁰) die Säuren, wie oben beschrieben, in inerten Lösemitteln in Anwesenheit einer Base und/oder eines Hilfsstoffes mit Verbindungen der allgemeinen Formel (V) 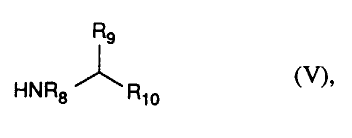 in welcher
R⁸, R⁹ und R¹⁰ die oben angegebene Bedeutung haben,
umsetzt,
und gegebenenfalls die Substituenten A, B, D und R¹ nach üblichen Methoden, beispielsweise durch Reduktion, Oxidation, Alkylierung oder Hydrolyse einführt oder in andere Gruppen überführt,
und gegebenenfalls die Isomeren trennt, und im Fall der Herstellung der Salze mit einer entsprechenden Base oder Säure umsetzt,

7. Arzneimittel enthaltend mindestens ein Imidazolyl-substituiertes Phenylessigsäureprolinamid nach Ansprüchen 1 bis 4.

8. Arzneimittel nach Anspruch 7 zur Behandlung von arterieller Hypertonie und Atherosklerose.

9. Verwendung von Imidazolyl-substituierten Phenylessigsäureprolinamiden nach Anspruch 1 bis 4 zur Herstellung von Arzneimitteln.

10. Verwendung nach Anspruch 9 zur Herstellung von Arzneimitteln zur Behandlung von arterieller Hypertonie und Atherosklerose.

## Claims

1. Imidazolyl-substituted phenylacetic acid prolinamides of the general formula 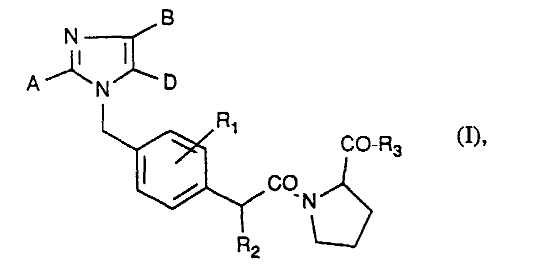 in which
A represents straight-chain or branched alkyl or alkenyl each having up to 8 carbon atoms, or represents cycloalkyl having 3 to 8 carbon atoms,
B represents hydrogen, halogen or perfluoroalkyl having up to 5 carbon atoms,
D represents a group of the formula -CH₂-OR⁴ or -CO-OR⁵,
in which
R⁴ denotes hydrogen or straight-chain or branched alkyl having up to 8 carbon atoms,
R⁵ denotes hydrogen, hydroxyl or straight-chain or branched alkoxy having up to 8 carbon atoms,
R¹ represents hydrogen, halogen, nitro, hydroxyl, trifluoromethyl, trifluoromethoxy, straight-chain or branched alkyl, alkoxy or alkoxycarbonyl each having up to 6 carbon atoms, cyano or carboxyl,
R² represents hydrogen, straight-chain or branched alkyl having up to 8 carbon atoms, or cycloalkyl having 3 to 8 carbon atoms,
R³ represents hydroxyl, benzyloxy or straight-chain or branched alkoxy having up to 8 carbon atoms, or
represents a group of the formula
-NR⁶R⁷ or 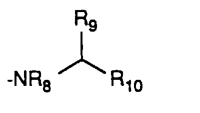 in which
R⁶, R⁷ and R⁸ are identical or different and denote hydrogen or straight-chain or branched alkyl having up to 8 carbon atoms,
or
R⁶ has the abovementioned meaning
and
R⁷ denotes a radical of the formula -SO₂R¹¹,
in which
R¹¹ denotes straight-chain or branched alkyl having up to 4 carbon atoms, which is optionally substituted by phenyl or cycloalkyl having 3 to 6 carbon atoms, or
denotes phenyl which is optionally substituted by straight-chain or branched alkyl having up to 4 carbon atoms,
R⁹ denotes phenyl which is optionally substituted up to 2 times by identical or different substituents from the series consisting of halogen, nitro, hydroxyl, trifluoromethyl, trifluoromethoxy, straight-chain or branched alkyl, alkoxy or alkoxycarbonyl each having up to 6 carbon atoms, cyano, carboxyl, cycloalkyl having 3 to 6 carbon atoms and phenyl,
R¹⁰ denotes carboxyl or straight-chain or branched alkoxycarbonyl having up to 8 carbon atoms, or
denotes a radical of the formula -CH₂OR¹², -CO-NR¹³R¹⁴ or -CH₂NR¹³R¹⁴,
in which
R¹² denotes hydrogen, benzyl or straight-chain or branched alkyl having up to 8 carbon atoms,
R¹³ and R¹⁴ are identical or different and denote hydrogen, phenyl or straight-chain or branched alkyl having up to 6 carbon atoms,
if appropriate in an isomeric form, and their salts.

2. Imidazolyl-substituted phenylacetic acid prolinamides of the formula (I) according to Claim 1, in which
A represents straight-chain or branched alkyl or alkenyl each having up to 6 carbon atoms, or represents cyclopropyl, cyclobutyl, cyclopentyl, cyclohexyl or cycloheptyl,
B represents hydrogen, fluorine, chlorine, bromine or perfluoroalkyl having up to 4 carbon atoms,
D represents a group of the formula -CH₂OR⁴ or -CO-R⁵,
in which
R⁴ denotes hydrogen or straight-chain or branched alkyl having up to 6 carbon atoms,
R⁵ denotes hydrogen, hydroxyl or straight-chain or branched alkoxy having up to 6 carbon atoms,
R¹ represents hydrogen, fluorine, chlorine, bromine, trifluoromethyl, cyano or straight-chain or branched alkyl, alkoxy or alkoxycarbonyl each having up to 5 carbon atoms,
R² represents hydrogen, straight-chain or branched alkyl having up to 6 carbon atoms, cyclopentyl, cyclohexyl or cycloheptyl,
R³ represents hydroxyl, benzyloxy or straight-chain or branched alkoxy having up to 6 carbon atoms, or represents a group of the formula
-NR⁶R⁷ or 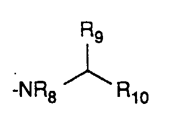 in which
R⁶, R⁷ and R⁸ are identical or different and denote hydrogen or straight-chain or branched alkyl having up to 6 carbon atoms,
or
R⁶ has the abovementioned meaning
and
R⁷ denotes a radical of the formula -SO₂R¹¹,
in which
R¹¹ denotes straight-chain or branched alkyl having up to 3 carbon atoms, benzyl or p-tolyl,
R⁹ denotes phenyl which is optionally substituted by fluorine, chlorine, bromine, trifluoromethyl, carboxyl or straight-chain or branched alkyl, alkoxy or alkoxycarbonyl each having up to 4 carbon atoms,
R¹⁰ denotes carboxyl or straight-chain or branched alkoxycarbonyl having up to 6 carbon atoms, or
denotes a radical of the formula -CH₂-OR¹², -CO-NR¹³R¹⁴ or -CH₂NR¹³R¹⁴,
in which
R¹² denotes hydrogen, benzyl or straight-chain or branched alkyl having up to 6 carbon atoms,
R¹³ and R¹⁴ are identical or different and denote hydrogen, phenyl or straight-chain or branched alkyl having up to 4 carbon atoms,
if appropriate in an isomeric form, and their salts.

3. Imidazolyl-substituted phenylacetic acid prolinamides of the formula (I) according to Claim 1, in which
A represents straight-chain or branched alkyl or alkenyl each having up to 4 carbon atoms, or represents cyclopropyl, cyclopentyl or cyclohexyl,
B represents hydrogen, fluorine, chlorine or perfluoroalkyl having up to 2 carbon atoms,
D represents a group of the formula -CH₂OR⁴ or -CO-R⁵,
in which
R⁴ denotes hydrogen or straight-chain or branched alkyl having up to 4 carbon atoms,
R⁵ denotes hydrogen, hydroxyl or straight-chain or branched alkoxy having up to 4 carbon atoms,
R¹ represents hydrogen, fluorine, chlorine, bromine, trifluoromethyl, trifluoromethoxy, methyl or isobutyl,
R² represents hydrogen, straight-chain or branched alkyl having up to 4 carbon atoms, cyclopentyl, cyclohexyl or cycloheptyl,
R³ represents hydroxyl, benzyloxy or straight-chain or branched alkoxy having up to 4 carbon atoms, or represents a group of the formula
-NR⁶R⁷ or 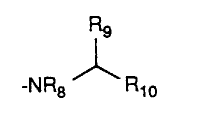 in which
R⁶, R⁷ and R⁸ are identical or different and denote hydrogen or straight-chain or branched alkyl having up to 4 carbon atoms,
or
R⁶ has the abovementioned meaning
and
R⁷ denotes a radical of the formula - SO₂R¹¹
in which
R¹¹ denotes methyl, benzyl or p-tolyl,
R⁹ denotes phenyl which is optionally substituted by fluorine, chlorine, bromine, trifluoromethyl, trifluoromethoxy or methyl,
R¹⁰ denotes carboxyl or straight-chain or branched alkoxycarbonyl having up to 4 carbon atoms, or denotes a radical of the formula -CH₂-OR¹², -CO-NR¹³R¹⁴ or -CH₂NR¹³R¹⁴,
in which
R¹² denotes hydrogen, benzyl or straight-chain or branched alkyl having up to 4 carbon atoms,
R¹³ and R¹⁴ are identical or different and denote hydrogen or straight-chain or branched alkyl having up to 3 carbon atoms,
if appropriate in an isomeric form, and their salts.

4. Imidazolyl-substituted phenylacetic acid prolinamides according to Claim 1, in which
R² represents cyclopentyl or cycloheptyl.

5. Imidazolyl-substituted phenylacetic acid prolinamides according to Claims 1 to 4 for therapeutic use.

6. Process for the preparation of imidazolyl-substituted phenylacetic acid prolinamides according to Claims 1 to 4, characterized in that
compounds of the general formula (II) 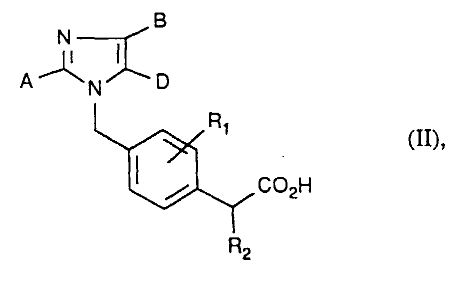 in which
A, B, D, R¹ and R² have the abovementioned meaning,
are reacted with proline derivatives of the general formula (III) 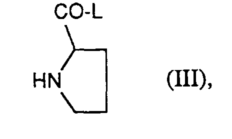 in which
L represents straight-chain or branched (C₁-C₄)-alkoxy or benzyloxy,
in inert solvents, in the presence of a base and/or of a dehydrating agent,
and in the case of the proline acids (R³ = OH), the esters are hydrolyzed,
and in the case of the prolinamides (R⁶/R⁷ = H or alkyl) starting from the acids, if appropriate after prior activation, reacted as described above with amines of the general formula (IV)
HNR⁶R⁷ (IV)
in which
R⁶ and R⁷ have the abovementioned meaning,
and in the case of the proline phenylglycinamides (-NR⁸-CHR⁹-R¹⁰) the acids are reacted, as described above, in inert solvents in the presence of a base and/or of an auxiliary with compounds of the general formula (V) 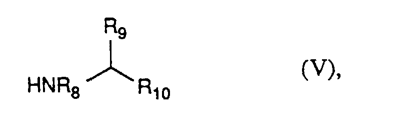 in which
R⁸, R⁹ and R¹⁰ have the abovementioned meaning,
and if appropriate the substituents A, B, D and R¹ are introduced or converted into other groups by customary methods, for example by reduction, oxidation, alkylation or hydrolysis,
and if appropriate the isomers are separated, and in the case of the preparation of the salts reacted with an appropriate base or acid.

7. Medicaments containing at least one imidazolyl-substituted phenylacetic acid prolinamide according to Claims 1 to 4.

8. Medicaments according to Claim 7 for the treatment of arterial hypertension and atherosclerosis.

9. Use of imidazolyl-substituted phenylacetic acid prolinamides according to Claims 1 to 4 for the production of medicaments.

10. Use according to Claim 9 for the production of medicaments for the treatment of arterial hypertension and atherosclerosis.

## Revendications

1. Prolinamides d'acide phénylacétique à substituant imidazolyle, de formule générale : 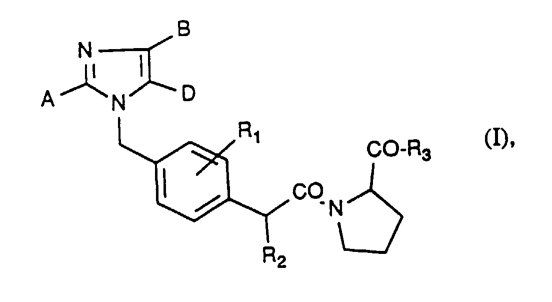 dans laquelle
A représente un groupe alkyle ou un groupe alcényle linéaire ou ramifié ayant chacun jusqu'à 8 atomes de carbone, ou bien un groupe cycloalkyle ayant 3 à 8 atomes de carbone,
B est de l'hydrogène, un halogène ou un groupe perfluoralkyle ayant jusqu'à 5 atomes de carbone,
D est un groupe de formule -CH₂-OR⁴ ou -CO-R⁵,
dans laquelle
R⁴ représente de l'hydrogène ou un groupe alkyle linéaire ou ramifié ayant jusqu'à 8 atomes de carbone,
R⁵ représente de l'hydrogène, un groupe hydroxy ou un groupe alkoxy linéaire ou ramifié ayant jusqu'à 8 atomes de carbone,
R¹ représente de l'hydrogène, un halogène, un groupe nitro, hydroxy, trifluorométhyle, trifluorométhoxy, un groupe alkyle, un groupe alkoxy ou un groupe alkoxycarbonyle linéaire ou ramifié ayant chacun jusqu'à 6 atomes de carbone, un groupe cyano ou carboxy,
R² représente de l'hydrogène, un groupe alkyle linéaire ou ramifié ayant jusqu'à 8 atomes de carbone ou un groupe cycloalkyle ayant 3 à 8 atomes de carbone,
R³ est un groupe hydroxy, benzyloxy ou un groupe alkoxy linéaire ou ramifié ayant jusqu'à 8 atomes de carbone, ou bien
un groupe de formule
-NR⁶R⁷ ou 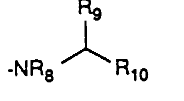 dans laquelle
R⁶, R⁷ et R⁸ sont identiques ou différents et représentent de l'hydrogène ou un groupe alkyle linéaire ou ramifié ayant jusqu'à 8 atomes de carbone,
ou bien
R⁶ a la définition indiquée ci-dessus
et
R⁷ est un reste de formule -SO₂R¹¹
dans laquelle
R¹¹ est un groupe alkyle linéaire ou ramifié ayant jusqu'à 4 atomes de carbone, qui est substitué le cas échéant par un radical phényle ou un radical cycloalkyle ayant 3 à 6 atomes de carbone, ou bien représente
un groupe phényle, qui est substitué le cas échéant par un radical alkyle linéaire ou ramifié ayant jusqu'à 4 atomes de carbone,
R⁹ désigne un groupe phényle qui est substitué le cas échéant jusqu'à deux fois identiques ou différentes par un halogène, un radical nitro, hydroxy, trifluorométhyle, trifluorométhoxy, un radical alkyle, un radical alkoxy ou un radical alkoxycarbonyle linéaire ou ramifié ayant chacun jusqu'à 6 atomes de carbone, un radical cyano, carboxy, cycloalkyle ayant 3 à 6 atomes de carbone ou un radical phényle,
R¹⁰ est un groupe carboxy ou un groupe alkoxy carbonyle linéaire ou ramifié ayant jusqu'à 8 atomes de carbone, ou bien un reste de formule -CH₂OR¹², -CO- NR¹³R¹⁴ ou -CH₂NR¹³R¹⁴, où
R¹² représente de l'hydrogène, un radical benzyle ou un radical alkyle linéaire ou ramifié ayant jusqu'à 8 atomes de carbone,
R¹³ et R¹⁴ sont identiques ou différents et représentent de l'hydrogène, un radical phényle ou un radical alkyle linéaire ou ramifié ayant jusqu'à 6 atomes de carbone,
le cas échéant sous une forme isomère, et leurs sels.

2. Prolinamides d'acide phénylacétique à substituant imidazolyle de formule (I) suivant la revendication 1, dans laquelle
A représente un groupe alkyle ou un groupe alcényle linéaire ou ramifié ayant chacun jusqu'à 6 atomes de carbone, ou bien
un groupe cyclopropyle, cyclobutyle, cyclopentyle, cyclohexyle ou cycloheptyle,
B est de l'hydrogène, du fluor, du chlore, du brome ou un groupe perfluoralkyle ayant jusqu'à 4 atomes de carbone,
D est un groupe de formule -CH₂-OR⁴ ou -CO-R⁵,
dans laquelle
R⁴ est de l'hydrogène ou un groupe alkyle linéaire ou ramifié ayant jusqu'à 6 atomes de carbone,
R⁵ est de l'hydrogène, un radical hydroxy ou un radical alkoxy linéaire ou ramifié ayant jusqu'à 6 atomes de carbone,
R¹ est de l'hydrogène, du fluor, du chlore, du brome, un groupe trifluorométhyle, cyano ou un groupe alkyle, un groupe alkoxy ou un groupe alkoxycarbonyle linéaire ou ramifié ayant chacun jusqu'à 5 atomes de carbone,
R² est de l'hydrogène, un groupe alkyle linéaire ou ramifié ayant jusqu'à 6 atomes de carbone, un groupe cyclopentyle, cyclohexyle ou cycloheptyle,
R³ est un groupe hydroxy, un groupe benzyloxy ou un groupe alkoxy linéaire ou ramifié ayant jusqu'à 6 atomes de carbone, ou bien
un groupe de formule
-NR⁶R⁷ ou 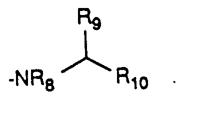 dans laquelle
R⁶, R⁷ et R⁸ sont identiques ou différents et représentent de l'hydrogène ou un groupe alkyle linéaire ou ramifié ayant jusqu'à 6 atomes de carbone,
ou bien
R⁶ a la définition indiquée ci-dessus
et
R⁷ est un reste de formule -SO₂R¹¹
dans laquelle
R¹¹ est un radical alkyle linéaire ou ramifié ayant jusqu'à 3 atomes de carbone, un radical benzyle ou p-tolyle,
R⁹ désigne un radical phényle qui est substitué le cas échéant par du fluor, du chlore, du brome, un radical trifluorométhyle, carboxy ou un radical alkyle, un radical alkoxy ou un radical alkoxycarbonyle linéaire ou ramifié ayant chacun jusqu'à 4 atomes de carbone,
R¹⁰ est un groupe carboxy ou un groupe alkoxy carbonyle linéaire ou ramifié ayant jusqu'à 6 atomes de carbone, ou bien un reste de formule -CH₂OR¹², -CO- NR¹³R¹⁴ ou -CH₂NR¹³R¹⁴,
où
R¹² représente de l'hydrogène, un radical benzyle ou un radical alkyle linéaire ou ramifié ayant jusqu'à 6 atomes de carbone,
R¹³ et R¹⁴ sont identiques ou différents et représentent de l'hydrogène, un radical phényle ou un radical alkyle linéaire ou ramifié ayant jusqu'à 4 atomes de carbone,
le cas échéant sous une forme isomère, et leurs sels.

3. Prolinamides d'acide phénylacétique à substituant imidazolyle de formule (I) suivant la revendication 1, dans laquelle
A est un groupe alkyle ou un groupe alcényle linéaire ou ramifié ayant chacun jusqu'à 4 atomes de carbone, ou bien
un groupe cyclopropyle, cyclopentyle ou cyclohexyle,
B est de l'hydrogène, du fluor, du chlore ou un groupe perfluoralkyle ayant jusqu'à 2 atomes de carbone,
D est un groupe de formule -CH₂-OR⁴ ou -CO-R⁵,
dans laquelle
R⁴ est de l'hydrogène ou un radical alkyle linéaire ou ramifié ayant jusqu'à 4 atomes de carbone,
R⁵ est de l'hydrogène, un radical hydroxy ou un radical alkoxy linéaire ou ramifié ayant jusqu'à 4 atomes de carbone,
R¹ est de l'hydrogène, du fluor, du chlore, du brome, un groupe trifluorométhyle, trifluorométhoxy, méthyle ou isobutyle,
R² est de l'hydrogène, un groupe alkyle linéaire ou ramifié ayant jusqu'à 4 atomes de carbone, un groupe cyclopentyle, cyclohexyle ou cycloheptyle,
R³ est un groupe hydroxy, benzyloxy ou un groupe alkoxy linéaire ou ramifié ayant jusqu'à 4 atomes de carbone, ou bien
un groupe de formule
-NR⁶R⁷ ou 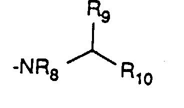 dans laquelle
R⁶, R⁷ et R⁸ sont identiques ou différents et représentent de l'hydrogène ou un radical alkyle linéaire ou ramifié ayant jusqu'à 4 atomes de carbone,
ou bien
R⁶ a la définition indiquée ci-dessus
et
R⁷ est un reste de formule -SO₂R¹¹
dans laquelle
R¹¹ est un radical méthyle, benzyle ou p-tolyle,
R⁹ est un radical phényle qui est substitué le cas échéant par du fluor, du chlore, du brome, un radical trifluorométhyle, trifluorométhoxy ou méthyle,
R¹⁰ est un groupe carboxy ou un groupe alkoxycarbonyle linéaire ou ramifié ayant jusqu'à 4 atomes de carbone, ou bien un reste de formule -CH₂OR¹², -CO- NR¹³R¹⁴ ou -CH₂NR¹³R¹⁴,
dans laquelle
R¹² est de l'hydrogène, un radical benzyle ou un radical alkyle linéaire ou ramifié ayant jusqu'à 4 atomes de carbone,
R¹³ et R¹⁴ sont identiques ou différents et représentent de l'hydrogène, un radical alkyle linéaire ou ramifié ayant jusqu'à 3 atomes de carbone,
le cas échéant sous une forme isomère, et leurs sels.

4. Prolinamides d'acide phénylacétique à substituant imidazolyle suivant la revendication 1,
dans lesquels
R² est un groupe cyclopentyle ou cycloheptyle.

5. Prolinamides d'acide phénylacétique à substituant imidazolyle suivant les revendications 1 à 4, destinés à une utilisation thérapeutique.

6. Procédé de production de prolinamides d'acide phénylacétique à substituant imidazolyle suivant les revendications 1 à 4, caractérisé en ce qu'on fait réagir des composés de formule générale (II) 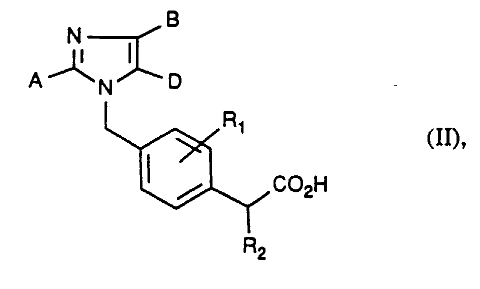 dans laquelle
A, B, D, R¹ et R² ont la définition indiquée ci-dessus, avec des dérivés de proline de formule générale (III) 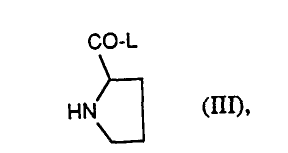 dans laquelle
L est un groupe alkoxy en C₁ à C₄ linéaire ou ramifié ou un groupe benzyloxy,
dans des solvants inertes, en présence d'une base et/ou d'un agent déshydratant,
et dans le cas des acides de proline (R³ = OH), on saponifie les esters,
et dans le cas des prolinamides (R⁶/R⁷ = H ou alkyle), on conduit la réaction à partir des acides, le cas échéant après une activation préalable, comme décrit ci-dessus, avec des amines de formule générale (IV),
HNR⁶R⁷ (IV),
dans laquelle R⁶ et R⁷ ont la définition indiquée ci-dessus, et dans le cas des phénylglycinamides de proline (-NR⁸-CHR⁹-R¹⁰), on fait réagir les acides, comme décrit ci-dessus, dans des solvants inertes, en présence d'une base et/ou d'une substance auxiliaire, avec des composés de formule générale (V), 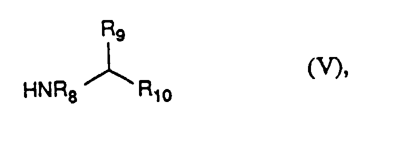 dans laquelle
R⁸, R⁹ et R¹⁰ ont la définition indiquée ci-dessus,
et le cas échéant, on introduit ou on transforme en d'autres groupes les substituants A, B, D et R¹ par des moyens classiques, par exemple par réduction, oxydation, alkylation ou hydrolyse,
et on sépare éventuellement les isomères, et dans le cas de la production des sels, on fait réagir avec une base correspondante ou un acide correspondant.

7. Médicament contenant au moins un prolinamide d'acide phénylacétique à substituant imidazolyle suivant les revendications 1 à 4.

8. Médicament suivant la revendication 7, destiné au traitement de l'hypertonie artérielle et de l'athérosclérose.

9. Utilisation de prolinamides d'acide phénylacétique à substituant imidazolyle suivant les revendications 1 à 4 pour la préparation de médicaments.

10. Utilisation suivant la revendication 9, pour la préparation de médicaments destinés au traitement de l'hypertonie artérielle et de l'athérosclérose.
